(19) 

**Europäisches Patentamt**
**European Patent Office**
**Office européen des brevets**

(11) **EP 3 954 992 A1**

(12) **EUROPEAN PATENT APPLICATION**

(43) Date of publication:
**16.02.2022 Bulletin 2022/07**

(21) Application number: **21172650.0**

(22) Date of filing: **04.05.2016**

(51) International Patent Classification (IPC):
**G01N 33/50** $^{(2006.01)}$  **G01N 33/574** $^{(2006.01)}$
**A61P 29/00** $^{(2006.01)}$  **A61P 35/00** $^{(2006.01)}$
**A61P 43/00** $^{(2006.01)}$  **G01N 33/94** $^{(2006.01)}$

(52) Cooperative Patent Classification (CPC):
**G01N 33/5011; A61P 29/00; A61P 35/00;**
**A61P 43/00; G01N 33/5014; G01N 33/5029;**
**G01N 33/5085;** G01N 33/948

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**

(30) Priority: **27.05.2015 US 201562166716 P**

(62) Document number(s) of the earlier application(s) in accordance with Art. 76 EPC:
**16799471.4 / 3 304 085**

(71) Applicant: **Cannabics Pharmaceuticals Inc**
**Bethesda, Maryland 20814 (US)**

(72) Inventor: **BALLAN, Eyal**
**Ramat Hasharon (IL)**

(74) Representative: **Lecomte & Partners**
**76-78, rue de Merl**
**2146 Luxembourg (LU)**

Remarks:
This application was filed on 07.05.2021 as a divisional application to the application mentioned under INID code 62.

(54) **METHOD FOR HIGH THROUGHPUT SCREENING OF CANCER CELLS**

(57) The present invention discloses a database of analytes, wherein said database comprises data concerning said analyte, correlated with a measurable effect on cells, defined by implementing personalized medicine PM based method comprising high throughput screening HTS for identifying antitumor effect of cannabis strains comprising the steps of: providing an array comprising a plurality of tumor cell samples of a patient's tumor biopsy; providing at least one cannabis extract of at least one strain to be tested; contacting said patient's tumor biopsy tumor cell samples with said at least one cannabis extract; and detecting a signal indicative of said antitumor effect relative to a control sample..

**FIG. 3C**

EP 3 954 992 A1

**Description**

FIELD OF THE INVENTION

The present disclosure relates to novel means and methods for high throughput screening of cancer cells. More particularly the current invention pertains to a method for high throughput screening (HTS) for identifying an analyte with a measurable effect on cells and a system thereof.

BACKGROUND OF THE INVENTION

Cannabinoids include phytocannabinoids, endogenous endocannabinoids, and synthetic cannabinoids. More than 60 phytocannabinoids have been identified within the Cannabis plant. Cannabinoids elicit their pharmacological activities through cannabinoid receptor type 1 (CB 1) and type 2 (CB2), two G-protein coupled receptors (GPCR) in the endocannabinoid signaling pathway. These receptors share 44% amino acid identity and a distinct yet similar binding profile for cannabinoids. CB1 receptors are found predominantly in the central and peripheral nervous systems and suppress neuronal excitability and transmitter release, leading to hypothermia, sedation, euphoria, and altered mental status. CB2 receptors are found at higher levels in the peripheral nervous system, gastrointestinal system and immune tissues.

Multiple sclerosis, neuropathic pain, cancer, atherosclerosis, stroke, myocardial infarction, hypertension, glaucoma, obesity/metabolic syndrome and osteoporosis are some of the diseases in which alterations in the cannabinoid pathway have been demonstrated. Since these diseases are found to be multifactorial, variations in expression and pharmacological cannabinoid receptor binding could be harnessed to elicit a therapeutic effect. Therefore, a defined botanical extract may better achieve this therapeutic goal than a single synthetic compound, as the multiple components could elicit a synergistic effect.

Cannabinoids are not yet approved for the treatment of cancer, although their anti-tumor effects have been known for over 30 years. Evidences exist that cannabinoids may have anti-cancer activity. This was noted in lung adenocarcinoma models in the 1970s and subsequent studies have demonstrated tumor growth inhibition *in vitro* and *in vivo* in glioblastoma, breast, prostate, thyroid, colon, skin, pancreatic, leukemia and lymphoma cancer cell models. The exact mechanism by which this anti-tumor effect occurs may involve suppression of proliferative cell signaling pathways, inhibition of angiogenesis and cell migration and induction of apoptosis and/or induction of autophagy.

A wide spectrum of cancer cells and mouse tumor models have been employed to evaluate the antitumor efficacy and the mechanisms of action of cannabinoids, supported by findings that the endocannabinoid system may be altered during various malignant and non-malignant disease states. Significant levels of cannabinoid receptors are found in prostate, breast, leukemia, melanoma, and thyroid cell lines, as well as colorectal and hepatocellular carcinoma tissue specimens. Of particular significance is the fact that in prostate cancer cell lines, the expression of both CB1 and CB2 is elevated compared to normal prostate cells. Similarly, in lymphoma and breast cancer tissue, as well as some derived cell lines, CB1 and CB2 are overexpressed.

It is reported that isolated compounds, which are then made or refined into synthetic drugs, are much more toxic than their plant sources. They produce effects of more rapid onset, greater intensity, and shorter duration. It is reported that they fail to reproduce the desirable effects of plants they come from.

Chemotherapy is an example of the attempt to cure a disease by producing a condition in the body that does not allow the disease to live or thrive. However, this therapy is unnatural and highly poisonous, and therefore, harmful.

Since cancer is a deadly disease people are whiling to suffer from harsh side effects, however, there is no biological link between the potency of therapy and its toxicity.

Personalized Medicine (PM) is a novel approach that proposes the customization of therapy being tailored to the individual patient. There are over 200 different known cancers and the genetic divergence among humans makes it nearly impossible to find one remedy for a group of people.

In view of the above, there is still a long felt and unmet need for novel therapeutic strategies for treating multifunctional diseases such as cancer, especially using botanical extracts.

SUMMARY OF THE INVENTION

It is therefore one object of the present invention to disclose a method for high throughput screening (HTS) for identifying an analyte with a measurable effect on cells, said method comprises steps of: (a) providing an array comprising a plurality of cell samples; (b) providing at least one analyte to be tested; (c) contacting said cell samples with said analyte; and (d) detecting a signal indicative of said measurable effect on cells, wherein alteration of said signal over time measured on said cell sample relative to a control sample, is indicative of said measurable effect of said analyte on said cell sample.

It is another object of the present invention to disclose the method as defined above, wherein said analyst is selected from the group consisting of cannabinoid-type, cannabinoid derivative, cannabis extract or fraction thereof, non cannabinoid-type constituent, product, compound, molecule or substance and any combination thereof.

It is a further object of the present invention to disclose the method as defined in any of the above, wherein said measurable effect on cells is selected from the group consisting of physiological, genetic, biochemical, structural and any combination thereof.

It is a further object of the present invention to disclose the method as defined in any of the above, wherein said measurable effect on cells is selected from the group consisting of: anti proliferative, regenerative, anti inflammatory, anti mitotic, differentiative, anti metastatic, anti angiogenic, apoptotic, cytotoxic, cytopathic and any combination thereof.

It is a further object of the present invention to disclose the method as defined in any of the above, wherein said measurable effect on cells is an effect on a biological parameter selected from the group consisting of: proliferation, migration, absorbance, adherence, apoptosis, necrosis, autophagy, cytotoxicity, cell size, motility, cell cycle and any combination thereof.

It is a further object of the present invention to disclose the method as defined in any of the above, wherein said cancer cells are selected from the group consisting of: breast, ovarian, colon/ rectum, prostate, melanoma, head and neck, pancreatic, osteosarcoma, gastric, glioma, glioblastoma, neuroblastoma, leukemia, adenocarcinoma, adrenal, anal, bile duct, bladder, bone, brain/CNS, cervical, endometrial, esophagus, eye, gastrointestinal, kidney, leukemia, liver, lung , lymphoma, multiple myeloma, nasal cavity and paranasal sinus, nasopharyngeal, non-hodgkin lymphoma, oral cavity, oropharyngeal, osteosarcoma, ovarian, pancreatic, penile, pituitary, retinoblastoma, rhabdomyosarcoma, salivary gland, sarcoma, skin, small intestine, stomach, testicular, thymus, thyroid, uterine sarcoma, vaginal and vulvar and any combination thereof.

It is a further object of the present invention to disclose the method as defined in any of the above, wherein said cell samples are selected from the group consisting of: xenografts, allografts, cell lines, biopsy cells and a combination thereof.

It is a further object of the present invention to disclose the method as defined in any of the above, wherein said cell lines are cancer cell lines selected from the group consisting of: central nervous system, bone, prostate, stomach, urinary tract, ovary, haematopoietic and lymphoid tissue, kidney, thyroid, skin, soft tissue, salivary gland, ovary, lung, pleura, liver, endometrium, pancreas, breast, upper aerodigestive tract, large intestine, autonomic ganglia, oesophagus, biliary tract, small intestine, autonomic ganglia and any combination thereof.

It is a further object of the present invention to disclose the method as defined in any of the above, wherein said cell samples are selected from the group consisting of: human cell lines, animal cell lines and xenografts.

It is a further object of the present invention to disclose the method as defined in any of the above, wherein said cell samples are selected from the group consisting of: cancer cells, stem cells, neuronal cells, cardiomyocyte cells, somatic cells germ cells, normal cells, and any combination thereof.

It is a further object of the present invention to disclose the method as defined in any of the above, wherein said signal is selected from the group consisting of: optic, luminescent, fluorescent, immunological, cell count, radioactive, non radioactive isotopic, electrical and any combination thereof.

It is a further object of the present invention to disclose the method as defined in any of the above, wherein said measurable effect on cells is an effect on the expression level of a cancer marker selected from the group consisting of: ALK gene, Alpha-fetoprotein (AFP), Beta-2-microglobulin (B2M), Beta-human chorionic gonadotropin (Beta-hCG), BCR-ABL fusion gene, BRAF mutation V600E, CA15-3/CA27.29, CA19-9, CA-125, Calcitonin, Carcinoembryonic antigen (CEA), CD20, Chromogranin A (CgA), Chromosomes 3, 7, 17, and 9p21, Cytokeratin fragments 21-1, EGFR mutation, Estrogen receptor (ER)/progesterone receptor (PR), Fibrin/fibrinogen, HE4, HER2/neu, Immunoglobulins, KIT, KRAS mutation, Lactate dehydrogenase, Nuclear matrix protein 22, Prostate-specific antigen (PSA), Thyroglobulin, Urokinase plasminogen activator (uPA), plasminogen activator inhibitor (PAI-1), 5-Protein signature (Oval), 21-Gene signature (Oncotype DX), 70-Gene signature (Mammaprint) and any combination thereof.

It is a further object of the present invention to disclose the method as defined in any of the above, wherein said analyte is extracted from cannabis; said cannabis is selected from a group consisting of: Cannabis sativa, Cannabis indica, Cannabis ruderalis, and any combination thereof.

It is a further object of the present invention to disclose the method as defined in any of the above, wherein said cannabinoid-type is selected from the group consisting of: Cannabigerol (CBG) type, Cannabichromene (CBC) type, Cannabidiol (CBD) type, Δ9 - Tetrahydrocannabinol (THC) type, Δ8 -THC type, Cannabicyclol (CBL) type, Cannabielsoin (CBE) type, Cannabinol (CBN) and Cannabinodiol (CBND) types, Cannabitriol (CBT) type, cannabinoids with miscellaneous types and any combination thereof.

It is a further object of the present invention to disclose the method as defined in any of the above, wherein said cannabinoid-type is further selected from the group consisting of: Tetrahydrocannabidiol (THC) or a derivative thereof, cannabidiol (CBD) or a derivative thereof, CBG (Cannabigerol), CBC (Cannabichromene), CBL (Cannabicyclol), CBV (Cannabivarin), THCV (Tetrahydrocannabivarin), CBDV (Cannabidivarin), CBCV (Cannabichromevarin), CBGV (Cannabigerovarin), CBGM (Cannabigerol Monomethyl Ether) and any combination thereof.

It is a further object of the present invention to disclose the method as defined in any of the above, wherein said THC or a derivative thereof is selected from the group consisting of THC, THCV, THCA, THCVA, Delta-9-tetrahydrocannabinol (Δ9-THC) and delta-8-tetrahydrocannabinol (Δ8-THC) and any combination thereof.

It is a further object of the present invention to disclose the method as defined in any of the above, wherein said cannabidiol (CBD) or a derivative thereof is selected from the group consisting of CBD, CBDV, CBDA and any combination thereof.

It is a further object of the present invention to disclose the method as defined in any of the above, wherein said non cannabinoid-type constituent, product, compound, molecule or substance is selected from the group consisting of: terpenoids, hydrocarbons, essential oil derived from cannabis, nitrogen-containing compounds, carbohydrates, flavonoids, fatty acids, amino acids, proteins, glycoproteins, enzymes, sugars and related compounds, noncannabinoid phenols, simple alcohols, aldehydes, ketones, acids, esters, lactones, steroids, terpenes, phytosterols such as campesterol, ergosterol, E-sitosterol, and stigmasterol, vitamins such as vitamin A and vitamin K, pigments such as carotene and xanthophylls, elements such as Na, K, Ca, Mg, Fe, Cu, Mn, Zn and Hg and any combination thereof.

It is a further object of the present invention to disclose the method as defined in any of the above, wherein said analyte is derived from a source selected from the group consisting of body of humans and animals, extracted from plants, synthetic, and any combination thereof.

It is a further object of the present invention to disclose the method as defined in any of the above, wherein said HTS is selected from the group consisting of: microtiter plate, automatic colony pickers, uHTS or ultra-high-throughput screening, 3D tumor spheroid analysis method for HTS drug discovery, Celigo Imaging Cytometer, automation systems, a carousel system to store assay plates for high storage capacity and high speed access, integrated robot system, readout or detection, data-collection process and any combination thereof.

It is a further object of the present invention to disclose the method as defined in any of the above, wherein said analyte provides a synergistic effect with respect to said measurable effect on cells as compared to the effect provided by conventional antitumor or anti- inflammatory therapies administered separately.

It is a further object of the present invention to disclose the method as defined in any of the above, wherein said analyte provides a contra indicatory effect with respect to antitumor or anti-inflammatory activity as compared to the effect provided by conventional antitumor or anti-inflammatory therapies administered separately.

It is a further object of the present invention to disclose a system for high throughput screening (HTS) for identifying an analyte with a measurable effect on cells, said system comprises: (a) an array comprising a plurality of cell samples; (b) at least one analyte to be tested; and (c) means for detecting a signal indicative of said measurable effect on cells, wherein alteration of said signal over time measured on said cell sample relative to a control sample, is indicative of said measurable effect of said analyte on said cell sample.

It is a further object of the present invention to disclose the system as defined above, wherein said analyst is selected from the group consisting of cannabinoid-type, cannabinoid derivative, cannabis extract or fraction thereof, non cannabinoid-type constituent, product, compound, molecule or substance and any combination thereof.

It is a further object of the present invention to disclose the system as defined in any of the above, wherein said measurable effect on cells is selected from the group consisting of physiological, genetic, biochemical, structural and any combination thereof.

It is a further object of the present invention to disclose the system as defined in any of the above, wherein said measurable effect on cells is selected from the group consisting of: anti proliferative, regenerative, anti inflammatory, anti mitotic, differentiative, anti metastatic, anti angiogenic, apoptotic, cytotoxic, cytopathic and any combination thereof.

It is a further object of the present invention to disclose the system as defined in any of the above, wherein said measurable effect on cells is an effect on a biological parameter selected from the group consisting of: proliferation, migration, absorbance, adherence, apoptosis, necrosis, autophagy, cytotoxicity, cell size, motility, cell cycle and any combination thereof.

It is a further object of the present invention to disclose the system as defined in any of the above, wherein said cancer cells are selected from the group consisting of: breast, ovarian, colon/ rectum, prostate, melanoma, head and neck, pancreatic, osteosarcoma, gastric, glioma, glioblastoma, neuroblastoma, leukemia, adenocarcinoma, adrenal, anal, bile duct, bladder, bone, brain/CNS, cervical, endometrial, esophagus, eye, gastrointestinal, kidney, leukemia, liver, lung , lymphoma, multiple myeloma, nasal cavity and paranasal sinus, nasopharyngeal, non-hodgkin lymphoma, oral cavity, oropharyngeal, osteosarcoma, ovarian, pancreatic, penile, pituitary, retinoblastoma, rhabdomyosarcoma, salivary gland, sarcoma, skin, small intestine, stomach, testicular, thymus, thyroid, uterine sarcoma, vaginal and vulvar and any combination thereof.

It is a further object of the present invention to disclose the system as defined in any of the above, wherein said cell samples are selected from the group consisting of: xenografts, allografts, cell lines, biopsy cells and a combination thereof.

It is a further object of the present invention to disclose the system as defined in any of the above, wherein said cell lines are cancer cell lines selected from the group consisting of: central nervous system, bone, prostate, stomach, urinary tract, ovary, haematopoietic and lymphoid tissue, kidney, thyroid, skin, soft tissue, salivary gland, ovary, lung, pleura, liver, endometrium, pancreas, breast, upper aerodigestive tract, large intestine, autonomic ganglia, oesophagus, biliary tract, small intestine, autonomic ganglia and any combination thereof.

It is a further object of the present invention to disclose the system as defined in any of the above, wherein said cell samples are selected from the group consisting of: human cell lines, animal cell lines and xenografts.

It is a further object of the present invention to disclose the system as defined in any of the above, wherein said cell samples are selected from the group consisting of: cancer cells, stem cells, neuronal cells, cardiomyocyte cells, somatic cells germ cells, normal cells, and any combination thereof.

It is a further object of the present invention to disclose the system as defined in any of the above, wherein said signal is selected from the group consisting of: optic, luminescent, fluorescent, immunological, cell count, radioactive, non radioactive isotopic, electrical and any combination thereof.

It is a further object of the present invention to disclose the system as defined in any of the above, wherein said measurable effect on cells is an effect on the expression level of a cancer marker selected from the group consisting of: ALK gene, Alpha-fetoprotein (AFP), Beta-2-microglobulin (B2M), Beta-human chorionic gonadotropin (Beta-hCG), BCR-ABL fusion gene, BRAF mutation V600E, CA15-3/CA27.29, CA19-9, CA-125, Calcitonin, Carcinoembryonic antigen (CEA), CD20, Chromogranin A (CgA), Chromosomes 3, 7, 17, and 9p21, Cytokeratin fragments 21-1, EGFR mutation, Estrogen receptor (ER)/progesterone receptor (PR), Fibrin/fibrinogen, HE4, HER2/neu, Immunoglobulins, KIT, KRAS mutation, Lactate dehydrogenase, Nuclear matrix protein 22, Prostate-specific antigen (PSA), Thyroglobulin, Urokinase plasminogen activator (uPA), plasminogen activator inhibitor (PAI-1), 5-Protein signature (Oval), 21-Gene signature (Oncotype DX), 70-Gene signature (Mammaprint) and any combination thereof.

It is a further object of the present invention to disclose the system as defined in any of the above, wherein said analyte is extracted from cannabis; said cannabis is selected from a group consisting of: Cannabis sativa, Cannabis indica, Cannabis ruderalis, and any combination thereof.

It is a further object of the present invention to disclose the system as defined in any of the above, wherein said cannabinoid-type is selected from the group consisting of: Cannabigerol (CBG) type, Cannabichromene (CBC) type, Cannabidiol (CBD) type, $\Delta9$ - Tetrahydrocannabinol (THC) type, $\Delta8$ -THC type, Cannabicyclol (CBL) type, Cannabielsoin (CBE) type, Cannabinol (CBN) and Cannabinodiol (CBND) types, Cannabitriol (CBT) type, cannabinoids with miscellaneous types and any combination thereof.

It is a further object of the present invention to disclose the system as defined in any of the above, wherein said cannabinoid-type is further selected from the group consisting of: Tetrahydrocannabidiol (THC) or a derivative thereof, cannabidiol (CBD) or a derivative thereof, CBG (Cannabigerol), CBC (Cannabichromene), CBL (Cannabicyclol), CBV (Cannabivarin), THCV (Tetrahydrocannabivarin), CBDV (Cannabidivarin), CBCV (Cannabichromevarin), CBGV (Cannabigerovarin), CBGM (Cannabigerol Monomethyl Ether) and any combination thereof.

It is a further object of the present invention to disclose the system as defined in any of the above, wherein said THC or a derivative thereof is selected from the group consisting of THC, THCV, THCA, THCVA, Delta-9-tetrahydrocannabinol ($\Delta9$-THC) and delta-8-tetrahydrocannabinol ($\Delta8$-THC) and any combination thereof.

It is a further object of the present invention to disclose the system as defined in any of the above, wherein said cannabidiol (CBD) or a derivative thereof is selected from the group consisting of CBD, CBDV, CBDA and any combination thereof.

It is a further object of the present invention to disclose the system as defined in any of the above, wherein said non cannabinoid-type constituent, product, compound, molecule or substance is selected from the group consisting of: terpenoids, hydrocarbons, essential oil derived from cannabis, nitrogen-containing compounds, carbohydrates, flavonoids, fatty acids, amino acids, proteins, glycoproteins, enzymes, sugars and related compounds, noncannabinoid phenols, simple alcohols, aldehydes, ketones, acids, esters, lactones, steroids, terpenes, phytosterols such as campesterol, ergosterol, E-sitosterol, and stigmasterol, vitamins such as vitamin A and vitamin K, pigments such as carotene and xanthophylls, elements such as Na, K, Ca, Mg, Fe, Cu, Mn, Zn and Hg and any combination thereof.

It is a further object of the present invention to disclose the system as defined in any of the above, wherein said analyte is derived from a source selected from the group consisting of body of humans and animals, extracted from plants, synthetic, and any combination thereof.

It is a further object of the present invention to disclose the system as defined in any of the above, wherein said HTS is selected from the group consisting of: microtiter plate, automatic colony pickers, uHTS or ultra-high-throughput screening, 3D tumor spheroid analysis method for HTS drug discovery, Celigo Imaging Cytometer, automation systems, a carousel system to store assay plates for high storage capacity and high speed access, integrated robot system, readout or detection, data-collection process and any combination thereof.

It is a further object of the present invention to disclose the system as defined in any of the above, wherein said analyte provides a synergistic effect with respect to said measurable effect on cells as compared to the effect provided by conventional antitumor or anti- inflammatory therapies administered separately.

It is a further object of the present invention to disclose the system as defined in any of the above, wherein said analyte provides a contra indicatory effect with respect to antitumor or anti-inflammatory activity as compared to the effect provided by conventional antitumor or anti-inflammatory therapies administered separately.

It is a further object of the present invention to disclose a non transitory computer readable medium comprising instructions which, when implemented by one or more computers cause the one or more computers to present data concerning a measurable effect on cells of one or more analytes on preselected cell samples by processing data concerning a signal indicative of said measurable effect on cells, wherein alteration of said signal over time measured on said cell sample relative to a control sample, is indicative of said measurable effect of said analyte on said cell sample.

It is a further object of the present invention to disclose a composition comprising therapeutically effective amount of, or an extract comprising essentially therapeutically effective amount of an analyte selected according to method of claim 1, wherein said composition has an antitumor or anti-inflammatory activity or synergistic effect thereof for use in the treatment of a cancer type or inflammatory disease.

It is a further object of the present invention to disclose the composition as defined above, wherein said analyst is selected from the group consisting of cannabinoid-type, cannabinoid derivative, cannabis extract or fraction thereof, non cannabinoid-type constituent, product, compound, molecule or substance and any combination thereof.

It is a further object of the present invention to disclose a method for identifying one or more genetic markers derived from cannabis, wherein said one or more genetic markers correlates with a measurable effect on cells as indicated by the method as defined in any of the above, said method comprises additional steps of correlating said signal with cannabis DNA sequence data.

It is a further object of the present invention to disclose one or more genetic markers derived from cannabis, wherein said one or more genetic markers correlates with a measurable effect on cells as indicated by the method as defined in any of the above, further wherein said signal is correlated with cannabis DNA sequence data.

It is a further object of the present invention to disclose the genetic markers as defined in any of the above, wherein said one or more genetic markers is selected from the group consisting of: variation, mutation or alteration in a genomic loci, a single nucleotide polymorphism (SNP), minisatellites, RFLP (Restriction fragment length polymorphism), SSLP (Simple sequence length polymorphism), AFLP (Amplified fragment length polymorphism), RAPD (Random amplification of polymorphic DNA), VNTR (Variable number tandem repeat), SSR (Simple sequence repeat), microsatellite polymorphism, STR (Short tandem repeat), SFP (Single feature polymorphism), DArT (Diversity Arrays Technology), RAD markers (Restriction site associated DNA markers) nucleotide changes, indel, deletion, duplication, inversion and/or insertion and any combination thereof.

It is a further object of the present invention to disclose a database of analytes, wherein said database comprises data concerning said analyte, correlated with a measurable effect on cells, defined by implementing steps as described in any of the above.

It is a further object of the present invention to disclose a system for high throughput screening (HTS) for identifying an analyte selected from the group consisting of: cannabis extract or a fraction thereof, cannabinoid-type constitute, non cannabinoid-type constitute and any combination thereof, said analyte is indicative of cytotoxic or anti proliferative or anti mitotic or cell growth inhibitory activity *in vitro*. The system comprises: (a) an array comprising a plurality of cancer cell samples; (b) at least one analyte to be tested, said analyte is selected from the group consisting of: cannabis extract or a fraction thereof, cannabinoid-type constitute, non cannabinoid-type constitute and any combination thereof; and (c) means for detecting a signal indicative of said cytotoxic or anti proliferative or anti mitotic or cell growth inhibitory activity *in vitro,* wherein alteration of said signal over time measured on said cancer cell sample relative to a control sample, is indicative of said cytotoxic or anti proliferative or anti mitotic or cell growth inhibitory activity *in vitro* of said analyte on said cancer cell sample.

It is a further object of the present invention to disclose a method for high throughput screening (HTS) for identifying an analyte selected from the group consisting of: cannabis extract or a fraction thereof, cannabinoid-type constitute, non cannabinoid-type constitute and any combination thereof, said analyte is indicative of cytotoxic or anti proliferative or anti mitotic or cell growth inhibitory activity *in vitro*. The method comprises steps of: (a) providing an array comprising a plurality of cancer cell samples; (b) providing said analyte to be tested, said analyte is selected from the group consisting of: cannabis extract or a fraction thereof, cannabinoid-type constitute, non cannabinoid-type constitute and any combination thereof; (c) contacting said cancer cell samples with said analyte; and (d) detecting a signal indicative of said cytotoxic or anti proliferative or anti mitotic or cell growth inhibitory activity *in vitro,* wherein alteration of said signal over time measured on said cancer cell sample relative to a control sample, is indicative of said cytotoxic or anti proliferative or anti mitotic or cell growth inhibitory activity *in vitro* of said analyte on said cancer cell sample.

It is a further object of the present invention to disclose a method for high throughput screening (HTS) for identifying an analyte selected from the group consisting of: cannabis extract or a fraction thereof, cannabinoid-type constitute, non cannabinoid-type constitute and any combination thereof, said analyte is indicative of antitumour activity, said method comprises steps of the method as defined in any of the above, and additionally comprising steps of: (a) transplanting cancer cell xenographs derived from said cancer cell samples into experimental animals; (b) treating said experimental animals with said analyte selected from the group consisting of: cannabis extract or a fraction thereof, cannabinoid-type constitute, non cannabinoid-type constitute and any combination thereof, identified by the method as defined in any of the above, and (c) monitoring tumor growth of said experimental animal.

It is a further object of the present invention to disclose the method as defined in any of the above, wherein said experimental animal is nude mice.

It is a further object of the present invention to disclose a protocol useful for identifying a botanical analyte with a measurable effect on cells correlated with a disease. The protocol comprises steps of: (a) providing input data comprising data selected from the group consisting of: parameters of said analyte, parameters of said cells, high throughput screening (HTS) results data for identifying an analyte with a measurable effect on cells as indicated in claim 1, clinical or preclinical data and any combination thereof; (b) processing said data; and (c) presenting output data at an electronic display concerning a measurable effects of said analyte on said cells correlated with a disease, and any combination thereof.

It is a further object of the present invention to disclose the protocol as defined above, wherein said data processing comprises steps selected from the group consisting of: correlating, normalizing, calibrating, factorizing, calculating, statistically analyzing and any combination thereof.

It is a further object of the present invention to disclose the protocol as defined in any of the above, wherein said analyte parameters are selected from the group consisting of: analyte source, analyte processing and any combination thereof.

It is a further object of the present invention to disclose the protocol as defined in any of the above, wherein said botanical analyte source parameters are selected from the group consisting of: source strain, source genotype, source phenotype, source growth conditions, source harvest conditions, source nutrition, source part or organ and any combination thereof.

It is a further object of the present invention to disclose the protocol as defined in any of the above, wherein said source part or organ is selected from the group consisting of: root, stem, leaf, flower, seed and any combination thereof.

It is a further object of the present invention to disclose the protocol as defined in any of the above, wherein said botanical analyte processing parameters are selected from the group consisting of: curing, drying, extraction process, decarboxylation and any combination thereof.

It is a further object of the present invention to disclose the protocol as defined in any of the above, wherein said extraction process is selected from the group consisting of: butane, $CO_2$ gradients, ethanol, dry ice and any combination thereof.

It is a further object of the present invention to disclose the protocol as defined in any of the above, wherein said cell parameters are selected from the group consisting of: cells source, cells treatment and any combination thereof.

It is a further object of the present invention to disclose the protocol as defined in any of the above, wherein said cells source is selected from the group consisting of: biopsies, cell lines, xenographs, mutated cells or molecules and any combination thereof.

It is a further object of the present invention to disclose the protocol as defined in any of the above, wherein said cells treatment is selected from the group consisting of: cells medium treatment, serum treatment, cells dilution, cell cycle phase and any combination thereof.

It is a further object of the present invention to disclose the protocol as defined in any of the above, wherein said measurable effect on cells is selected from the group consisting of proliferation, apoptosis, migration, regeneration, differentiation, angiogenesis, and any combination thereof.

It is a further object of the present invention to disclose the protocol as defined in any of the above, wherein said clinical or preclinical data is selected from the group consisting of: administration route of said analyte to a subject, dosages, release form, cancer markers level, tumor size monitoring, metastasis monitoring, survival, quality of life measured according to one or more scales, and any combination thereof.

It is a further object of the present invention to disclose the protocol as defined in any of the above, wherein said administration route is selected from the group consisting of: sublingual, oral, intravenous, topical, subcutaneous and any combination thereof.

It is a further object of the present invention to disclose the protocol as defined in any of the above, wherein said release form is selected from the group consisting of: slow release, controlled release, sustained release, immediate or rapid release and any combination thereof.

It is a further object of the present invention to disclose the protocol as defined in any of the above, wherein said cancer markers are selected from the group consisting of: ALK gene, Alpha-fetoprotein (AFP), Beta-2-microglobulin (B2M), Beta-human chorionic gonadotropin (Beta-hCG), BCR-ABL fusion gene, BRAF mutation V600E, CA15-3/CA27.29, CA19-9, CA-125, Calcitonin, Carcinoembryonic antigen (CEA), CD20, Chromogranin A (CgA), Chromosomes 3, 7, 17, and 9p21, Cytokeratin fragments 21-1, EGFR mutation, Estrogen receptor (ER)/progesterone receptor (PR), Fibrin/fibrinogen, HE4, HER2/neu, Immunoglobulins, KIT, KRAS mutation, Lactate dehydrogenase, Nuclear matrix protein 22, Prostate-specific antigen (PSA), Thyroglobulin, Urokinase plasminogen activator (uPA), plasminogen activator inhibitor (PAI-1), 5-Protein signature (Oval), 21-Gene signature (Oncotype DX), 70-Gene signature (Mammaprint) and any combination thereof.

It is a further object of the present invention to disclose the protocol as defined in any of the above, wherein said one or more scales for assessing quality of life are selected from the group consisting of: pain scale, quality of life scale, functional assessment of cancer therapy scale and any combination thereof.

BRIEF DESCRIPTION OF THE FIGURES

Exemplary non-limited embodiments of the disclosed subject matter will be described, with reference to the following description of the embodiments, in conjunction with the figures. The figures are generally not shown to scale and any sizes are only meant to be exemplary and not necessarily limiting. Corresponding or like elements are optionally designated by the same numerals or letters.

Fig. 1 is presenting a test report for cannabinoid analysis;

Fig. 2 is a photographic illustration of an imaging device ImageXpress micro;

Fig. 3 is presenting images of cells analysed using MetaXpress and the Cell Health module;

Fig. 3A is presenting 3 different filters imaging Hoechst 33342 (blue), YO-PRO-1 (green) and PI (red);

Fig. 3B is showing segmentation of the cells, according to intensity of fluorescent markers by the Cell Health module;

Fig. 3C is an example of screen display showing measurements of late apoptotic cells in each well;

Fig. 4 is presenting images of different cancer cell lines treated with selected cannabis extracts as compared to control;

Fig. 5 is presenting images of prostate cancer cell lines treated with various cannabis extracts as compared to control;

Fig. 6 is presenting images of glioblastoma cell lines treated with selected cannabis extracts comprising various THC to CBD ratios;

Fig. 7 is graphically presenting the effect of five different cannabis extracts on three human cancer cell line types;

Fig. 8 is presenting images showing the effect of selected cannabis extracts on PC3 prostate carcinoma cells;

Fig. 9 is presenting a scheme illustrating a protocol for identifying a botanical analyte with a measurable effect on cells correlated with a disease.

DETAILED DESCRIPTION OF THE PREFERRED EMBODIMENTS

In the following detailed description of the preferred embodiments, reference is made to the accompanying drawings that form a part hereof, and in which are shown by way of illustration specific embodiments in which the invention may be practiced. It is understood that other embodiments may be utilized and structural changes may be made without departing from the scope of the present invention. The present invention may be practiced according to the claims without some or all of these specific details. For the purpose of clarity, technical material that is known in the technical fields related to the invention has not been described in detail so that the present invention is not unnecessarily obscured.

The present invention provides a method for high throughput screening (HTS) for identifying an analyte with a measurable effect on cells. The aforementioned method comprises steps of: (a) providing an array comprising a plurality of cell samples; (b) providing at least one analyte to be tested; (c) contacting said cell samples with said analyte and (d) detecting a signal indicative of said measurable effect on cells, wherein alteration of said signal over time measured on said cell sample relative to a control sample, is indicative of said measurable effect of said analyte on said cell sample.

According to a specific aspect, the present invention provides a method for high throughput screening (HTS) for identifying an analyte selected from the group consisting of: cannabis extract or a fraction thereof, cannabinoid-type, non cannabinoid-type and any combination thereof. It is within the scope that the analyte is indicative of cytotoxic or anti proliferative or anti mitotic or cell growth inhibitory activity *in vitro*. Such a method comprises steps of: (a) providing an array comprising a plurality of cancer cell samples; (b) providing said analyte to be tested, said analyte is selected from the group consisting of: cannabis extract or a fraction thereof, cannabinoid-type , non cannabinoid-type and any combination thereof; (b) contacting said cancer cell samples with said analyte; (c) detecting a signal indicative of said cytotoxic or anti proliferative or anti mitotic or cell growth inhibitory activity *in vitro,* wherein alteration of said signal over time measured on said cancer cell sample relative to a control sample, is indicative of said cytotoxic or anti proliferative or anti mitotic or cell growth inhibitory activity *in vitro* of said analyte on said cancer cell sample.

As used herein the term **"about"** denotes $\pm$ 25% of the defined amount or measure or value.

The term **"high throughput screening"** or **"HTS"** used hereinafter refers to any method for scientific experimentation especially used in the fields of biology and chemistry. The screening facility includes usage of robotics, data processing and control software, liquid handling devices, and sensitive detectors allowing a researcher to quickly conduct millions of chemical, genetic, or pharmacological tests. Through this process one can rapidly identify active compounds or analytes, antibodies, or genes that modulate a particular biomolecular pathway. The results of these experiments provide starting points for drug design and for understanding the interaction or role of a particular biochemical process in biology.

It is herein acknowledged that automation is an important element in HTS's technique. Typically, an integrated robot system consisting of one or more robots transports assay-microplates from station to station for sample and reagent addition, mixing, incubation, and finally readout or detection. An HTS system can usually prepare, incubate, and analyze many samples simultaneously, further speeding the data-collection process. It is further within the scope that the term HTS further relates to uHTS or ultra-high-throughput screening referring to screening in excess of 100,000 compounds per day.

It is further within the scope that additional or HTS methods are used in the present invention such as 3D tumor spheroid analysis method for HTS drug discovery using Celigo Imaging Cytometer, automation systems such as a carousel system to store assay plates for high storage capacity and high speed access and any other HTS system or technique.

The term **"analyte"** as used hereinafter generally refers to a component, a molecule, a substance or chemical or botanical constituent that is of interest in an analytical procedure. The analytical procedure is designed to measure properties of the analyte.

The term **"cannabis"** refers hereinafter to a genus of flowering plants that includes three different species, Cannabis sativa, Cannabis indica and Cannabis ruderalis.

It is within the scope that cannabis extract or cannabis concentrates or fractions thereof are used as analytes on cell samples for screening for a measurable effect on cells. Such an extract may include cannabinoid- type compounds or fractions, non-cannabinoid- type compounds or fractions and combinations thereof.

The term **"non cannabinoid"** or **"non cannabinoid-type"** as used hereinafter refers to any molecule or compound or constitute which is not a cannabinoid.

The term **"Cannabinoids"** refer hereinafter to a class of diverse chemical compounds that act on cannabinoid receptors and other signal transduction receptors or proteins on cells that repress or activate neurotransmitter release in the brain, heart, liver, immune system and lungs. These receptor proteins include the endocannabinoids (produced naturally in the body by humans and animals), the phytocannabinoids (found in cannabis and some other plants), and synthetic cannabinoids (manufactured chemically). The most notable cannabinoid is the phytocannabinoid Δ9-tetrahydrocannabinol (THC), the primary psychoactive compound of cannabis. Cannabidiol (CBD) is another major constituent of the plant, representing up to 40% in extracts of the plant resin. There are at least 85 different cannabinoids isolated from cannabis, exhibiting varied effects. Reference is now made to http://www.medicinalgenomics.com/wp-content/uploads/2011/12/Chemical-constituents-of-cannabis.pdf, which is incorporated herein by reference in its entirety, presenting a non limiting list of identified cannabinoids. The current invention includes all cannabinoids, for example, cannabinoids belonging to the following classes or groups:

- Cannabigerol (CBG) type: including CBG, and its precursor cannabigerolic acid (CBGA) shown to be a biogenic cannabinoid formed in the plant. Propyl side-chain analogs and a monomethyl ether derivative are other cannabinoids of this group.
- Cannabichromene (CBC) type: Five CBC-type cannabinoids, mainly present as C5-analogs, have been identified.
- Cannabidiol (CBD) type: Seven CBD-type cannabinoids with C1 to C5 side chains have been described. CBD and its corresponding acid CBDA are the most abundant cannabinoids in fiber-type Cannabis (industrial hemp). CBDA was the first discovered cannabinoid acid.
- Δ9 -Tetrahydrocannabinol (THC) type: Nine THC-type cannabinoids with C1 to C5 side chains are known. The major biogenic precursor is the THC acid A, whereas THC acid B is present to a much lesser extent. THC is the main psychotropic principle; the acids are not psychoactive. THC (6a,10a-trans-6a,7,8,10a-tetrahydro-6,6,9- trimethyl-3-pentyl-6H-dibenzo[b,d]pyran-1-ol) are also included within this group.
- Δ8 -THC type: Δ8 -THC and its acid precursor are considered as THC and THC acid artifacts, respectively. The 8,9 double-bond position is thermodynamically more stable than the 9,10 position. Δ8 -THC is approx 20% less active than THC.

- Cannabicyclol (CBL) type: Three cannabinoids characterized by a five-atom ring and C1-bridge instead of the typical ring A are known: CBL, its acid precursor, and the C3 side-chain analog. CBL is known to be a heat-generated artifact from CBC.
- Cannabielsoin (CBE) type: Among the five CBE-type cannabinoids, which are artifacts formed from CBD, are CBE and its acid precursors A and B.
- Cannabinol (CBN) and Cannabinodiol (CBND) types: Six CBN- and two CBND-type cannabinoids are known. With ring A aromatized, they are oxidation artifacts of THC and CBD, respectively. Their concentration in Cannabis products depends on age and storage conditions.
- Cannabitriol (CBT) type: Nine CBT-type cannabinoids have been identified, which are characterized by additional OH substitution. CBT itself exists in the form of both isomers and the racemate, whereas two isomers (9-a- and 9-b-hydroxy) of CBTV were identified. CBDA tetrahydrocannabitriol ester (ester at 9-hydroxy group) is the only reported ester of any naturally occurring cannabinoids.
- Miscellaneous types: Eleven cannabinoids of various unusual structure, e.g., with a furano ring (dehydrocannabifuran, cannabifuran), carbonyl function (cannabichromanon, 10- oxo-G-6a-tetrahydrocannabinol), or tetrahydroxy substitution (cannabiripsol), are known.

The term **"cannabinoid extract"** refers hereinafter to any extract or concentrate derived from the cannabis plant which contains at least one cannabinoid. The cannabinoids may be extracted from the cannabis plant using any one of the many known extraction methods, such as non-hydrocarbons extraction methods and hydrocarbons extraction methods.

The term **"cannabinoid fraction"** used hereinafter refers to cannabis extract treated by separation or purification or fractionation processes. More particularly it refers to purified or partially purified cannabis extract containing cannabinoid-type portions or elements. In alternative embodiments, cannabinoid fraction may contain synthetic cannabinoids.

The term **"cannabidiol" or "CBD"** refers hereinafter to one of at least 85 active cannabinoids identified in cannabis.Cannabidiol is a major phytocannabinoid, accounting for up to 40% of the plant's extract. CBD is considered to have a wider scope of medical applications than tetrahydrocannabidiol (THC). Cannabidiol has a very low affinity for CB1 and CB2 receptors but acts as an indirect antagonist of their agonists. CBD may potentiate THC's effects by increasing CB1 receptor density or through another CB1-related mechanism. It is also an inverse agonist of CB2 receptors. CBD possesses antiproliferative, pro-apoptotic effects and inhibits cancer cell migration, adhesion and invasion. The term CBD also refers to Cannabidivarin (CBDV) a homolog of cannabidiol (CBD) and to cannabidiolic acid (CBDA).

The term **"Tetrahydrocannabidiol" or "THC"** refers hereinafter to the principal psychoactive constituent (or cannabinoid) of the cannabis plant. THC has a partial agonist activity at the cannabinoid receptor CB1, and the CB2 receptor and is known to increase cortisol levels. It is further included within the scope that the term THC further refers to Tetrahydrocannabivarin (THCV or THV) a homologue of tetrahydrocannabinol (THC) and Tetrahydrocannabinolic acid (THCA, 2-COOH-THC), a biosynthetic precursor of tetrahydrocannabinol (THC).

It is noted that cannabinol (CBN), cannabichromene (CBC), the acids (CBDA, CBGA, THCA) and propyl homologues (CBDV, CBGV, THCV) of CBD, cannabigerol (CBG) and THC, and tetrahydrocannabivarin acid (THC-V and THC-VA) are also included as optional active ingredient(s) of the composition or formulation of the present invention.

The cannabis extract or a fraction thereof may comprise noncannabinoid-type constituents selected from the group consisting of: terpenoids, hydrocarbons, essential oil derived from cannabis, nitrogen-containing compounds, carbohydrates, flavonoids, fatty acids, noncannabinoid phenols, simple alcohols, aldehydes, ketones, acids, esters, lactones, phytosterols such as campesterol, ergosterol, E-sitosterol, and stigmasterol, vitamin K, pigments such as carotene and xanthophylls, elements such as Na, K, Ca, Mg, Fe, Cu, Mn, Zn and Hg and any combination thereof. Reference is made to the publication of http://www.medicinalgenomics.com/wp-content/uploads/2011/12/Chemical-constituents-of-cannabis.pdf, which is incorporated herein by reference in its entirety. It is further within the scope that there are 483 different identifiable chemical constituents known to exist in cannabis. The most distinctive and specific class of compounds are the cannabinoids (66 known), that are only known to exist in the cannabis plant. Other constituents of the cannabis plant are: nitrogenous compounds (27 known), amino acids (18), proteins (3), glycoproteins (6), enzymes (2), sugars and related compounds (34), hydrocarbons (50), simple alcohols (7), aldehydes (13), ketones (13), simple acids (21), fatty acids (22), simple esters (12), lactones (1), steroids (11), terpenes (120), non-cannabinoid phenols (25), flavonoids (21), vitamins (1) [Vitamin A], pigments (2), and elements (9). It is further within the scope that http://medicalmarijuana.procon.org/view.answers.php?questionID=000636 is incorporated herein in its entirety.

The term **"cannabinoid receptor"** refers hereinafter to a class of cell membrane receptors under the G protein-coupled receptor superfamily. There are currently two known subtypes of cannabinoid receptors, termed CB1 and CB2. The CB1 receptor is expressed mainly in the brain, but also in the lungs, liver and kidneys. The CB2 receptor is expressed mainly in the immune system, the digestive system and in hematopoietic cells.

It is further within the scope that cell lines screened by the method and system of the present invention include, but are not limited to the list of cell lines detailed in http://www.broadinstitute.org/ccle/data/browseSamples?actionMethod=pages%2Fsearch%2F searchResult.xhtml%3AbrowseSamplesBean.checkSkipFirstStep%28%29&conversation-Prop agation=begin, incorporated herein by reference.

The term **"quality of life scale"** as used hereinafter refers to scales for assessing quality of life of a patient after treatment. Non limiting examples of such scales include pain scales such as Faces Pain Scale, Wong-Baker FACES Pain Rating Scale, Coloured Analogue Scale, Visual Analog Scale (VAS), Verbal Numerical Rating Scale (VNRS), Verbal Descriptor Scale (VDS) and Brief Pain Inventory; Quality of Life Scale (QOLS); functional assessment of cancer therapy (FACT) scale and any combination thereof.

The term **"sustained release dosage form"** refers hereinafter to the release of a drug at a predetermined rate in order to maintain a constant drug concentration for a specific period of time with minimum side effects. This can be achieved through a variety of formulations, including liposomes and drug-polymer conjugates. Sustained release's definition is more akin to a "controlled release" rather than "sustained".

According to certain embodiments, the present invention provides a personalized medicine (PM) based system and method for screening for novel cancer therapies which comprises at least one of the following aspects:

1. High Through output Screening (HTS) of cells derived from patients' biopsies treated with cannabinoid extracts.
2. High Through output Screening (HTS) of cells derived from patients' biopsies treated with cannabinoid fractions.
3. High Through output Screening (HTS) of biopsies derived xenogarphs (i.e. mice injected with human cancer cells) treated with cannabinoid fractions.
4. High Through output Screening (HTS) of biopsies derived xenogarphs (i.e. mice injected with human cancer cells) treated with cannabinoid extract.
5. High Through output Screening (HTS) of cells derived from patients' biopsies treated with cannabinoid extracts and conventional chemotherapy.
6. High Through output Screening (HTS) of specific cancer cell lines.
7. Correlating patients' biological data (Genetic/blood/neurology/behavior/nutrition) with data from HTS on biopsies.
8. Correlating patients' Clinical data with HTS data.

It is therefore one object of the present invention to provide a method and system for screening for nontoxic natural cancer therapy.

Up until now, the common concept of the drug industry is to use isolates, or to synthesize, parts of the whole plant, which, during use by medical practitioners, sometimes produces undesirable side effects in their patients. For instance, the most powerful drug used in cancer chemotherapy was isolated from the plant Madagascar periwinkle. It is an effective agent against breast and lymph cancers. However, its side effects may be debilitating and dangerous. It is therefore noted that the isolation of active molecules from plants may be miscalculated. In certain cases, a single compound could not account for desirable properties of plant extract or a fraction thereof. The assumption that it would be better to treat a disease with a purified compound rather than with the whole plant extract may be misleading.

It is herein acknowledged that it has been shown that using the whole cannabis plant extract may be more effective in treating certain diseases and conditions relative to isolated compounds derived from cannabis. There may be synergistic or additive effects between the various cannabis extract components which will be absent when using isolated compounds or specific combinations thereof.

According to a further aspect, without wishing to be bound by theory, harmonized ratios of active molecules within mixtures of extracts (biological mechanisms) may be the result of co-evolution along with human receptors.

The continues scanning and the building of big data for an "oriented evolution of active ratios" is a further unmet need, realized by the present invention.

Without wishing to be bound by theory, it is a well-established prospect that the state of mind affects the physiology of our body through its balance that is experienced as "health" and its imbalance that is experienced as a "disease". Most of today's cancer therapies are harmful and toxic regardless of their therapeutic benefit. It is plausible that this phenomenon is related to the cultural way of thinking that a harsh disease is cured by a harsh treatment. However, on real grounds, cancer patients are usually "sick" people with a minor pain that become "treated" patients with unbearable pain. Nausea and weight loss weakens the body immensely and derives the will to live. The pessimistic prophecy of the proximity of death in conjunction with the weakened body is a high barrier to cross to become healthy again. Therefore, empowering the emotional state of cancer patients is translated into the physiological realm. The current invention pertains to providing a treatment that addresses both the psychological state of cancer patients as well as their physiological condition. While cannabinoids or cannabis extracts are screened *inter alia* for their cytotoxic or anticancer properties it is also their "side effects" that are desirable since they are well known as beneficial for Cancer Related Cachexia and Anorexia Syndrome. They are also known for pain reduction and antidepressant activity. These combined therapeutic benefits make the current invention a potent therapy with minimal undesirable side effects.

Using high screening technology for biological processes such as cell necrosis and apoptosis in cannabinoid treated cancer cell lines and /or cells from tumors derived from patients, preferable natural cannabinoid combinations are herein identified.

It is further within the scope of the present invention to screen for and provide potent extract which is found to halt cancer cells to be administered to patients in a predetermined dosage form or administration rout such as capsule, intravenously or orally.

According to a further aspect, treating a cancer patient with an extract (i.e. cannabis extract or a fraction thereof) identified by the method of the present invention, benefits the following therapeutic prospects:

-   Uplifting the state of mind of the patient

-   No harmful side effects

-   An individually tailored highly potent anticancer compound

-   Improving appetite and reduce nausea regardless of anticancer properties of the extract, in cases where the patient is additionally treated with chemotherapy or radiation.

According to a further aspect of the invention, pharmacological importance of cannabinoids in cancer and other non-malignant diseases is revealed by the current invention.

To address the objectives and aspects disclosed above, the present invention provides a robust procedure of high through output screening (HTS) for the detection of correlations between selected analytes such as cannabinoid ratios or dosages, and anti-tumor activity. According to one embodiment, the present invention uses a growing library of human cancer cell lines tumor cells derived from patients or experimental animals, and creating an enlarged variety of cannabis-based compounds. Examination of the biological activity of these compounds on tumor cells of distinct tissue lineage creates a highly potent therapeutic data.

It is further within the scope that the HTS system is applied on cell lines, for the screening for potent cannabinoid or other natural extracts, with or without the conjunction of standard chemotherapy.

It is further within the scope that the HTS system is applied on biopsies derived from patients for the screening for potent cannabinoid or cannabinoid combinations or other natural extracts with the conjunction of chemotherapy according to patients' overall treatment.

It is further within the scope that a learning algorithm to predict cannabinoid ratio or terpens or mixtures or extracts potencies is developed by the present invention.

In another embodiment, a bank of tumor specific highly effective proprietary compounds is provided.

According to a further aspect, the effect of cannabinoids or cannabis extracts or a fraction thereof or other analyte of interest is assessed on cancer cells, stem cells, neurons and cardiomyocyte cells for the development of advanced natural therapeutics.

The antitumor activity of cannabinoids reflected in their potent therapeutic activity against diverse types of cancers is assessed by the system and method of the present invention.

In order to identify active compounds such as cannabinoid-type compound or cannabinoid ratios with an effect on specific types of cancer cells, the present invention provides a screen (i.e. high throughput screen, or HTS), which tests the anti-tumor activity of different cannabis derived fractions. The antitumor activity tests include: anti-proliferative effects (cell cycle arrest), decreased viability and cell death measured by cytotoxicity colorimetric assays such as XTT assays, apoptosis, necrosis, autophagy, as well as anti-angiogenic, anti-migratory, and anti-metastatic assays and possible synergetic effects of cannabinoids with conventional chemotherapeutic drugs that are currently in clinical use.

According to one embodiment, in order to perform the screen, the ImageXpress Micro XLS System is used. The ImageXpress Micro XLS System is a wide-field automated microscope capable of fluorescent, transmitted light, and phase-contrast imaging of fixed- or live cell studies. This state-of-the art system has the capability to collect a fewer images per well. It has a shorter imaging time with a field of view that is three times larger than industry current standards. Moreover, the ImageXpress Micro XLS System can capture > 10 million cells/hour in a low-resolution, whole-well, 3-color cell scoring application, or > 1 million cells/hour in a high resolution two-color assay which will be used to perform a high-throughput screening (HTS). From this HTS screen parameters of cell size, proliferation, apoptosis and migration are obtained. Additionally, current studies describe that the cannabinoids exerted selective anti-tumor activity in several distinct tumor models. The present invention is capable of rapidly and effectively screening many human and mouse cancer cell lines including: breast, ovarian, colon, prostate, melanoma, head and neck, pancreatic, osteosarcoma, gastric, glioma, glioblastoma, neuroblastoma, leukemia and more. Moreover in certain aspects of the invention, the effect of the tested compound or analyte is simultaneously tested on cancer cells, normal cells, metastatic cells and/ or to tumor cells after chemotherapeutic treatment and relapse derived from the same tissue and/or from the same patient.

It is therefore, within the scope of the present invention to provide a method for high throughput screening (HTS) for identifying an analyte with a measurable effect on cells. The aforementioned method comprises steps of: (a) providing an array comprising a plurality of cell samples; (b) providing at least one analyte to be tested; (c) contacting the cell samples with the analyte; and (d) detecting a signal indicative of the measurable effect on cells, wherein alteration of the signal over time measured on the cell sample relative to a control sample, is indicative of the measurable effect of the analyte on the cell sample.

It is further within the scope to disclose the method as defined in any of the above, wherein the analyst is selected from the group consisting of cannabinoid-type, cannabinoid derivative, cannabis extract or fraction thereof, non cannabinoid-type constituent, product, compound, molecule or substance and any combination thereof.

It is further within the scope to disclose the method as defined in any of the above, wherein the measurable effect on cells is selected from the group consisting of physiological, genetic, biochemical, structural and any combination thereof.

It is further within the scope to disclose the method as defined in any of the above, wherein the measurable effect on cells is selected from the group consisting of: anti proliferative, regenerative, anti inflammatory, anti mitotic, differentiative, anti metastatic, anti angiogenic, apoptotic, cytotoxic, cytopathic and any combination thereof.

It is further within the scope to disclose the method as defined in any of the above, wherein the measurable effect on cells is an effect on a biological parameter selected from the group consisting of: proliferation, migration, absorbance, adherence, apoptosis, necrosis, autophagy, cytotoxicity, cell size, motility, cell cycle and any combination thereof.

It is further within the scope to disclose the method as defined in any of the above, wherein the cancer cells are selected from the group consisting of: breast, ovarian, colon/ rectum, prostate, melanoma, head and neck, pancreatic, osteosarcoma, gastric, glioma, glioblastoma, neuroblastoma, leukemia, adenocarcinoma, adrenal, anal, bile duct, bladder, bone, brain/CNS, cervical, endometrial, esophagus, eye, gastrointestinal, kidney, leukemia, liver, lung , lymphoma, multiple myeloma, nasal cavity and paranasal sinus, nasopharyngeal, non-hodgkin lymphoma, oral cavity, oropharyngeal, osteosarcoma, ovarian, pancreatic, penile, pituitary, retinoblastoma, rhabdomyosarcoma, salivary gland, sarcoma, skin, small intestine, stomach, testicular, thymus, thyroid, uterine sarcoma, vaginal and vulvar and any combination thereof. It is further within the scope to disclose the method as defined in any of the above, wherein the cell samples are selected from the group consisting of: xenografts, allografts, cell lines, biopsy cells and a combination thereof.

It is further within the scope to disclose the method as defined in any of the above, wherein the cell lines are cancer cell lines selected from the group consisting of: central_nervous_system, bone, prostate, stomach, urinary_tract, ovary, haematopoietic_and_lymphoid_tissue, kidney, thyroid, skin, soft tissue, salivary_gland, ovary, lung, pleura, liver, endometrium, pancreas, breast, upper_aerodigestive tract, large intestine, autonomic_ganglia, oesophagus, biliary tract, small_intestine, autonomic_ganglia and any combination thereof.

It is further within the scope to disclose the method as defined in any of the above, wherein the cell samples are selected from the group consisting of: human cell lines, animal cell lines and xenografts.

It is further within the scope to disclose the method as defined in any of the above, wherein the cell samples are selected from the group consisting of: cancer cells, stem cells, neuronal cells, cardiomyocyte cells, somatic cells germ cells, normal cells, and any combination thereof.

It is further within the scope to disclose the method as defined in any of the above, wherein the signal is selected from the group consisting of: optic, luminescent, fluorescent, immunological, cell count, radioactive, non radioactive isotopic, electrical and any combination thereof.

It is further within the scope to disclose the method as defined in any of the above, wherein the measurable effect on cells is an effect on the expression level of a cancer marker selected from the group consisting of: ALK gene, Alpha-fetoprotein (AFP), Beta-2-microglobulin (B2M), Beta-human chorionic gonadotropin (Beta-hCG), BCR-ABL fusion gene, BRAF mutation V600E, CA15-3/CA27.29, CA19-9, CA-125, Calcitonin, Carcinoembryonic antigen (CEA), CD20, Chromogranin A (CgA), Chromosomes 3, 7, 17, and 9p21, Cytokeratin fragments 21-1, EGFR mutation, Estrogen receptor (ER)/progesterone receptor (PR), Fibrin/fibrinogen, HE4, HER2/neu, Immunoglobulins, KIT, KRAS mutation, Lactate dehydrogenase, Nuclear matrix protein 22, Prostate-specific antigen (PSA), Thyroglobulin, Urokinase plasminogen activator (uPA), plasminogen activator inhibitor (PAI-1), 5-Protein signature (Oval), 21-Gene signature (Oncotype DX), 70-Gene signature (Mammaprint) and any combination thereof.

It is further within the scope to disclose the method as defined in any of the above, wherein the analyte is extracted from cannabis; the cannabis is selected from a group consisting of: Cannabis sativa, Cannabis indica, Cannabis ruderalis, and any combination thereof.

It is further within the scope to disclose the method as defined in any of the above, wherein the cannabinoid-type is selected from the group consisting of: Cannabigerol (CBG) type, Cannabichromene (CBC) type, Cannabidiol (CBD) type, $\Delta 9$ -Tetrahydrocannabinol (THC) type, $\Delta 8$ -THC type, Cannabicyclol (CBL) type, Cannabielsoin (CBE) type, Cannabinol (CBN) and Cannabinodiol (CBND) types, Cannabitriol (CBT) type, cannabinoids with miscellaneous types and any combination thereof.

It is further within the scope to disclose the method as defined in any of the above, wherein the cannabinoid-type is further selected from the group consisting of: Tetrahydrocannabidiol (THC) or a derivative thereof, cannabidiol (CBD) or a derivative thereof, CBG (Cannabigerol ), CBC (Cannabichromene), CBL (Cannabicyclol), CBV (Cannabivarin), THCV (Tetrahydrocannabivarin), CBDV (Cannabidivarin), CBCV (Cannabichromevarin), CBGV (Cannabigerovarin), CBGM (Cannabigerol Monomethyl Ether) and any combination thereof.

It is further within the scope to disclose the method as defined in any of the above, wherein the THC or a derivative thereof is selected from the group consisting of THC, THCV, THCA, THCVA, Delta-9-tetrahydrocannabinol (Δ9-THC) and delta-8-tetrahydrocannabinol (Δ8-THC) and any combination thereof.

It is further within the scope to disclose the method as defined in any of the above, wherein the cannabidiol (CBD) or a derivative thereof is selected from the group consisting of CBD, CBDV, CBDA and any combination thereof.

It is further within the scope to disclose the method as defined in any of the above, wherein the non cannabinoid-type constituent, product, compound, molecule or substance is selected from the group consisting of: terpenoids, hydrocarbons, essential oil derived from cannabis, nitrogen-containing compounds, carbohydrates, flavonoids, fatty acids, amino acids, proteins, glycoproteins, enzymes, sugars and related compounds, noncannabinoid phenols, simple alcohols, aldehydes, ketones, acids, esters, lactones, steroids, terpenes, phytosterols such as campesterol, ergosterol, E-sitosterol, and stigmasterol, vitamins such as vitamin A and vitamin K, pigments such as carotene and xanthophylls, elements such as Na, K, Ca, Mg, Fe, Cu, Mn, Zn and Hg and any combination thereof.

It is further within the scope to disclose the method as defined in any of the above, wherein the analyte is derived from a source selected from the group consisting of body of humans and animals, extracted from plants, synthetic, and any combination thereof.

It is further within the scope to disclose the method as defined in any of the above, wherein the HTS is selected from the group consisting of: microtiter plate, automatic colony pickers, uHTS or ultra-high-throughput screening, 3D tumor spheroid analysis method for HTS drug discovery, Celigo Imaging Cytometer, automation systems, a carousel system to store assay plates for high storage capacity and high speed access, integrated robot system, readout or detection, data-collection process and any combination thereof.

It is further within the scope to disclose the method as defined in any of the above, wherein the analyte provides a synergistic effect with respect to the measurable effect on cells as compared to the effect provided by conventional antitumor or anti- inflammatory therapies administered separately.

It is further within the scope to disclose the method as defined in any of the above, wherein the analyte provides a contra indicatory effect with respect to antitumor or anti-inflammatory activity as compared to the effect provided by conventional antitumor or anti-inflammatory therapies administered separately.

It is further within the scope to disclose a system for high throughput screening (HTS) for identifying an analyte with a measurable effect on cells. The aforementioned system comprises: (a) an array comprising a plurality of cell samples; (b) at least one analyte to be tested; and (c) means for detecting a signal indicative of the measurable effect on cells, wherein alteration of the signal over time measured on the cell sample relative to a control sample, is indicative of the measurable effect of the analyte on the cell sample.

It is further within the scope to provide a non transitory computer readable medium comprising instructions which, when implemented by one or more computers cause the one or more computers to present data concerning a measurable effect on cells of one or more analytes on preselected cell samples by processing data concerning a signal indicative of the measurable effect on cells, wherein alteration of the signal over time measured on the cell sample relative to a control sample, is indicative of the measurable effect of the analyte on the cell sample.

It is further within the scope to provide a composition comprising therapeutically effective amount of, or an extract comprising essentially therapeutically effective amount of an analyte selected as defined by the method described in any of the above, wherein the composition has an antitumor or anti-inflammatory activity or synergistic effect thereof for use in the treatment of a cancer type or inflammatory disease.

It is further within the scope to provide a method for identifying one or more genetic markers derived from cannabis, wherein the one or more genetic markers correlates with a measurable effect on cells as indicated by the method as defined in any of the above, the method comprises additional steps of correlating the signal with cannabis DNA sequence data.

It is further within the scope to disclose provide one or more genetic markers derived from cannabis, wherein the one or more genetic markers correlates with a measurable effect on cells as indicated by the method as defined in any of the above, further wherein the signal is correlated with cannabis DNA sequence data.

It is further within the scope to disclose the method as defined in any of the above, , wherein the one or more genetic markers is selected from the group consisting of: variation, mutation or alteration in a genomic loci, a single nucleotide polymorphism (SNP), minisatellites, RFLP (Restriction fragment length polymorphism), SSLP (Simple sequence length polymorphism), AFLP (Amplified fragment length polymorphism), RAPD (Random amplification of polymorphic DNA), VNTR (Variable number tandem repeat), SSR (Simple sequence repeat), microsatellite polymorphism, STR (Short tandem repeat), SFP (Single feature polymorphism), DArT (Diversity Arrays Technology), RAD markers (Restriction site associated DNA markers) nucleotide changes, indel, deletion, duplication, inversion and/or insertion and any combination thereof.

It is further within the scope to provide a database of analytes, wherein the database comprises data concerning the analyte, correlated with a measurable effect on cells, defined by implementing steps of the method described in any of the above.

It is further within the scope to provide a method for high throughput screening (HTS) for identifying an analyte selected from the group consisting of: cannabis extract or a fraction thereof, cannabinoid-type constitute, non cannabinoid-type constitute and any combination thereof, the analyte is indicative of cytotoxic or anti proliferative or anti mitotic or cell growth inhibitory activity *in vitro*. The method comprises steps of: (a) providing an array comprising a plurality of cancer cell samples; (b) providing the analyte to be tested, the analyte is selected from the group consisting of: cannabis extract or a fraction thereof, cannabinoid-type constitute, non cannabinoid-type constitute and any combination thereof; (c) contacting the cancer cell samples with the analyte; and (d) detecting a signal indicative of the cytotoxic or anti proliferative or anti mitotic or cell growth inhibitory activity *in vitro,* wherein alteration of the signal over time measured on the cancer cell sample relative to a control sample, is indicative of the cytotoxic or anti proliferative or anti mitotic or cell growth inhibitory activity *in vitro* of the analyte on the cancer cell sample.

It is further within the scope to provide a method for high throughput screening (HTS) for identifying an analyte selected from the group consisting of: cannabis extract or a fraction thereof, cannabinoid-type constitute, non cannabinoid-type constitute and any combination thereof, the analyte is indicative of antitumour activity, the method comprises steps of the method as described in any of the above, and additionally comprising steps of: (a) transplanting cancer cell xenographs derived from the cancer cell samples into experimental animals; (b) treating the experimental animals with the analyte selected from the group consisting of: cannabis extract or a fraction thereof, cannabinoid-type constitute, non cannabinoid-type constitute and any combination thereof, identified by the method as defined in any of the above, and (c) monitoring tumor growth of the experimental animal.

It is further within the scope to disclose the method as defined in any of the above, wherein the experimental animal is nude mice.

It is further within the scope to provide a protocol useful for identifying a botanical analyte with a measurable effect on cells correlated with a disease. The aforementioned protocol comprises steps of: (a) providing input data comprising data selected from the group consisting of: parameters of the analyte, parameters of the cells, high throughput screening (HTS) results data for identifying an analyte with a measurable effect on cells as indicated in claim 1, clinical or preclinical data and any combination thereof; (b) processing the data; and (c) presenting output data at an electronic display concerning a measurable effects of the analyte on the cells correlated with a disease, and any combination thereof.

It is further within the scope to disclose the protocol as defined in any of the above, wherein the data processing comprises steps selected from the group consisting of: correlating, normalizing, calibrating, factorizing, calculating, statistically analyzing and any combination thereof.

It is further within the scope to disclose the protocol as defined in any of the above, wherein the analyte parameters are selected from the group consisting of: analyte source, analyte processing and any combination thereof.

It is further within the scope to disclose the protocol as defined in any of the above, wherein the botanical analyte source parameters are selected from the group consisting of: source strain, source genotype, source phenotype, source growth conditions, source harvest conditions, source nutrition, source part or organ and any combination thereof.

It is further within the scope to disclose the protocol as defined in any of the above, wherein the source part or organ is selected from the group consisting of: root, stem, leaf, flower, seed and any combination thereof.

It is further within the scope to disclose the protocol as defined in any of the above, wherein the botanical analyte processing parameters are selected from the group consisting of: curing, drying, extraction process, decarboxylation and any combination thereof.

It is further within the scope to disclose the protocol as defined in any of the above, wherein the extraction process is selected from the group consisting of: butane, $CO_2$ gradients, ethanol, dry ice and any combination thereof.

It is further within the scope to disclose the protocol as defined in any of the above, wherein the cell parameters are selected from the group consisting of: cells source, cells treatment and any combination thereof.

It is further within the scope to disclose the protocol as defined in any of the above, wherein the cells source is selected from the group consisting of: biopsies, cell lines, xenographs, mutated cells or molecules and any combination thereof.

It is further within the scope to disclose the protocol as defined in any of the above, wherein the cells treatment is selected from the group consisting of: cells medium treatment, serum treatment, cells dilution, cell cycle phase and any combination thereof.

It is further within the scope to disclose the protocol as defined in any of the above, wherein the measurable effect on cells is selected from the group consisting of proliferation, apoptosis, migration, regeneration, differentiation, angiogenesis, and any combination thereof.

It is further within the scope to disclose the protocol as defined in any of the above, wherein the clinical or preclinical data is selected from the group consisting of: administration route of the analyte to a subject, dosages, release form, cancer markers level, tumor size monitoring, metastasis monitoring, survival, quality of life measured according to one or more scales, and any combination thereof.

It is further within the scope to disclose the protocol as defined in any of the above, wherein the administration route is selected from the group consisting of: sublingual, oral, intravenous, topical, subcutaneous and any combination thereof.

It is further within the scope to disclose the protocol as defined in any of the above, wherein the release form is selected from the group consisting of: slow release, controlled release, sustained release, immediate or rapid release and any combination thereof.

It is further within the scope to disclose the protocol as defined in any of the above, wherein the cancer markers are selected from the group consisting of: ALK gene, Alpha-fetoprotein (AFP), Beta-2-microglobulin (B2M), Beta-human chorionic gonadotropin (Beta-hCG), BCR-ABL fusion gene, BRAF mutation V600E, CA15-3/CA27.29, CA19-9, CA-125, Calcitonin, Carcinoembryonic antigen (CEA), CD20, Chromogranin A (CgA), Chromosomes 3, 7, 17, and 9p21, Cytokeratin fragments 21-1, EGFR mutation, Estrogen receptor (ER)/progesterone receptor (PR), Fibrin/fibrinogen, HE4, HER2/neu, Immunoglobulins, KIT, KRAS mutation, Lactate dehydrogenase, Nuclear matrix protein 22, Prostate-specific antigen (PSA), Thyroglobulin, Urokinase plasminogen activator (uPA), plasminogen activator inhibitor (PAI-1), 5-Protein signature (Oval), 21-Gene signature (Oncotype DX), 70-Gene signature (Mammaprint) and any combination thereof.

It is further within the scope to disclose the protocol as defined in any of the above, wherein the one or more scales for assessing quality of life are selected from the group consisting of: pain scale, quality of life scale, functional assessment of cancer therapy scale and any combination thereof.

In order to understand the invention and to see how it may be implemented in practice, a plurality of preferred embodiments will now be described, by way of non-limiting example only, with reference to the following examples.

EXAMPLE 1

**A protocol for cannabis extraction**

Dried flowers of six cannabis sativa strains (i.e. CNB1, CNB2, CNB3, CNB4, CNB6, CNB8) were soaked in butane and resin was purged to exclude butane residues from the concentrated oil.

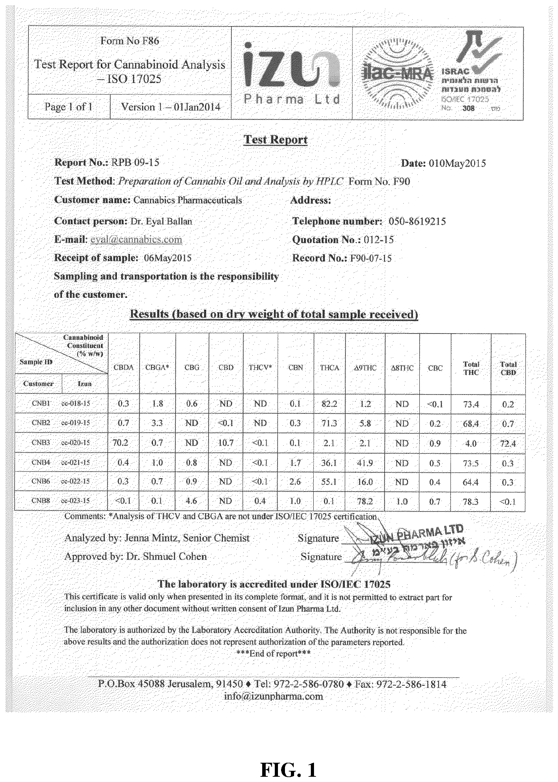

The concentration of 10 cannabinoids (i.e. CBDA, CBGA, CBG, CBD, THCV, CBN, THCA, Δ9THC, Δ8THC and CBC) in the extracts were evaluated in HPLC (see **Fig. 1**).

For preparation of a stock for cell lines, the cannabis extracts were dissolved in DMSO (e.g. about 50 mg/ml) and kept in -20°C until use.

EXAMPLE 2

**A protocol for cell line preparation**

Reference is now made to non limiting examples of cell cultures used in the present invention:

Examples of human cancer cell lines:

- MDA-MB-231 and MCF-7 breast carcinoma cells

- U87MG and U118MG glioblastoma cells

- PC3 prostate carcinoma cells

- HT29 and SW480 colon carcinoma cells

- AGS gastric adenocarcinoma cells

- MiaPaCa2 pancreatic carcinoma cells

Examples of human non-cancer cell lines (Control):

- Stabilized non-tumor cell lines HDF (human dermal fibroblasts)

- HaCat (human keratinocytes)

Reference is now made to a procedure for growing cell lines:
Cell lines were maintained at 37°C in a humidified atmosphere containing 5% $CO_2$.

All cell lines, except for PC3 and SW480 were routinely grown in phenol red-containing minimum essential medium (DMEM) (Sigma). PC3 and SW480 were grown in phenol red containing RPMI-1640 (Sigma).

Media were supplemented with 10% fetal bovine serum (FBS), 100 U/mL of penicillin, 100 $\mu$g/mL of streptomycin and 100mM L-glutamine.

Reference is now made to cell viability studies:

Cells were harvested three days before exposure and seeded (about 50,000-200,000 cells/well) into a 24-well microplate. The medium was replaced in respective culture medium containing 0.5% FBS, and vehicle (DMSO) or cannabis extracts (1-10 $\mu$g/ml) were added to the medium for 24- 48 hours in duplicates.

The effect of cannabinoid compounds on cell viability was measured using high-content screening analysis. For example, differentiation of subpopulations of cells within the same well was analysed by distinguishing: live versus necrotic cells, early apoptotic cells and late apoptotic cells.

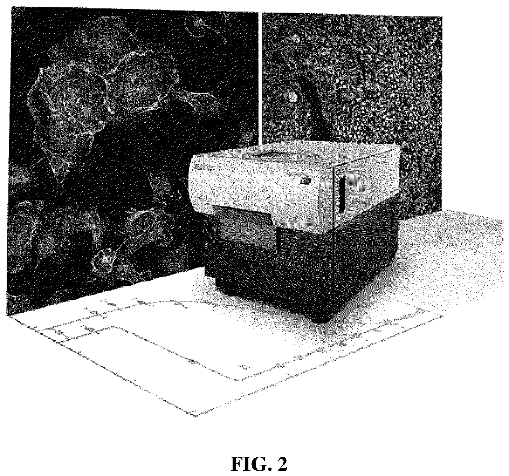

Cells were imaged in ImageXpress micro (see **Fig. 2**), and analyzed using MetaXpress and the Cell Cycle module.

Reference is now made to non limiting examples of probes used to screen the effect of cannabis extract on tested cell lines:
After predetermined periods of incubation, the cell lines medium was replaced with PBS containing fluorescent probes, including at least one of:

- Hoechst 33342, a UV-excitable blue fluorescent probe, stains the condensed chromatin of apoptotic cells and more dimly stains the normal chromatin of live cells.
- YO-PRO-1, green fluorescent dye which can enter apoptotic cells.
- Propidium Iodide (PI), red-fluorescent dye which cannot enter apoptotic cells.

It is noted that the staining pattern resulting from the simultaneous use of these three dyes makes it possible to distinguish normal, apoptotic and dead cell populations by flow cytometry or fluorescence microscopy.

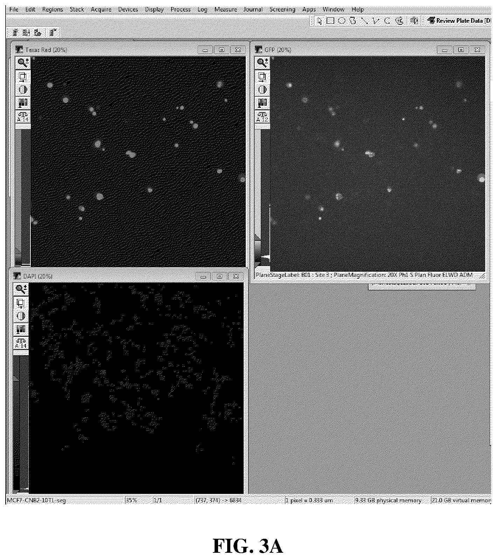

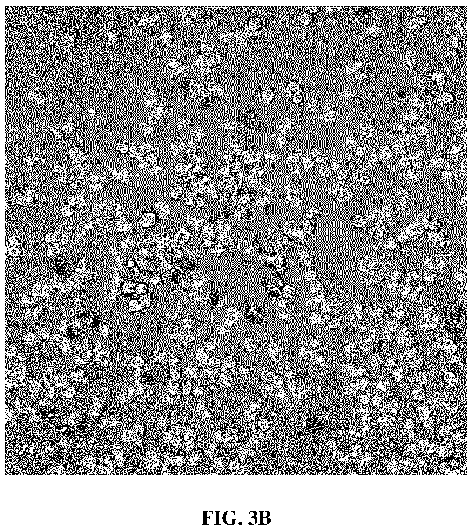

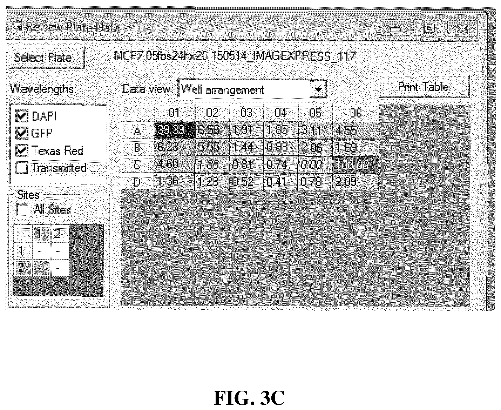

Reference is now made to **Fig. 3** presenting images of cells analysed using MetaXpress and the Cell Health module. In **Fig. 3A,** 3 different filters imaging Hoechst 33342 (blue), YO-PRO-1 (green) and PI (red) are presented. In **Fig. 3B,** it is shown that segmentation of the cells, according to intensity of fluorescent markers by the Cell Health module, differentiate subpopulation of cells: viable cells (green), early apoptotic cells (blue) and late apoptotic cells (pink) overlayed on transmitted light image. **Fig. 3C,** presents an example of measurements of number of late apoptotic cells in each well.

EXAMPLE 3

**Antitumor activity of cannabis extracts on cancer cell lines *in vitro***

Objective:

Providing a robust procedure and system for high through output screening (HTS) for the detection of correlations between cannabinoid ratios and dosages, and anti-tumor activity. The procedure includes screening of a growing library of human cancer cell lines and/or biopsies by an enlarged variety of cannabis-based compounds or extracts. It is demonstrated by the present invention that the examination of the biological activity of cannabis extracts, fractions and compounds thereof on tumor cell lines or biopsies of distinct tissue lineage, creates a highly potent therapeutic data. In another aspect, the HTS system and method is applied on cell lines for the screening of potent cannabinoids, cannabis fraction or extract, with or without the conjunction of standard chemotherapy. In another embodiment, the HTS system and method is applied on biopsies derived from patients, for the screening of most potent cannabinoid or cannabis extracts or fractions thereof with or without the conjunction of chemotherapy according to patients' overall treatment.

Results:

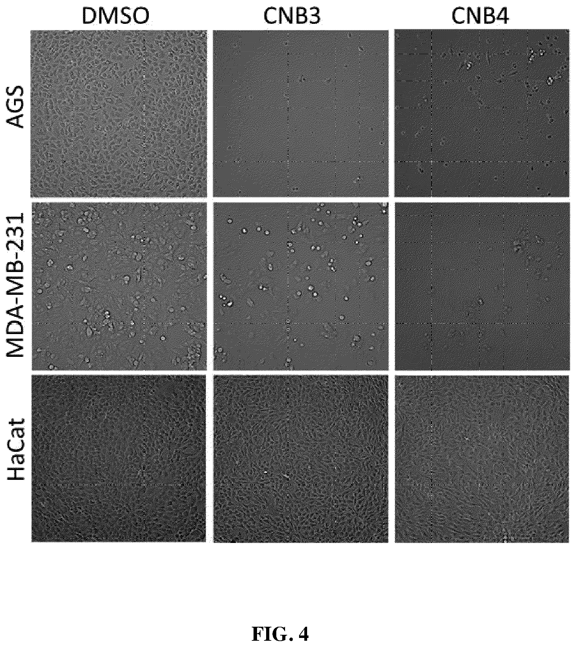

Reference is now made to **Fig. 4** showing images of different cancer cell lines treated with selected cannabis extracts as compared to control. In this experiment, 200,000 cells/well of AGS gastric adenocarcinoma cells, MDA-MB-231 breast carcinoma cells and HaCat human keratinocytes were plated into a 24-well microplate and treated with vehicle (DMSO) or with cannabis extracts (10 μg/ml) of two different strains (i.e. CNB3 and CNB4). Cells were imaged after 24 hours using the ImageXpress micro. It is shown in **Fig. 4** that both cannabis extracts (i.e. CNB3 and CNB4) dramatically reduced cell viability in cancer cell lines but not in normal human keratinocytes. This results show the specificity of the antitumor activity of the cannabis extracts and their usefulness in treating cancer cells. It is further shown that treating the cell lines with vehicle (i.e. DMSO) as a control, did not affect cell viability.

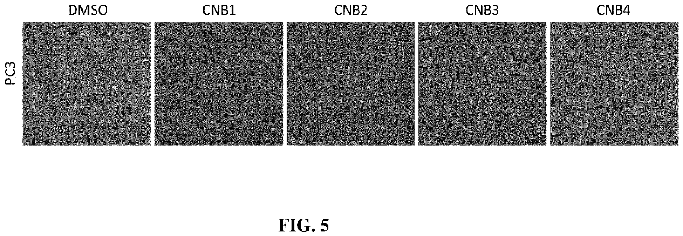

Reference is now made to **Fig. 5** showing images of prostate cancer cell lines treated with various cannabis extracts as compared to control. In this experiment, PC3 prostate carcinoma cells were plated into a 24-well microplate and treated with vehicle (DMSO) or cannabis extracts (10 μg/ml). Cells were imaged after 24 hours using the ImageXpress micro. The results presented in Fig.5 clearly demonstrate the effect of various cannabis extracts on PC3 prostate carcinoma cells. In this figure, CNB1 and CNB2 extracts demonstrate the most potent effect on prostate cell lines.

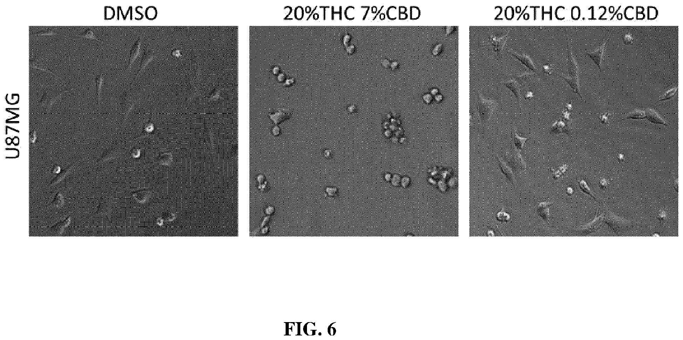

Reference is now made to **Fig. 6** showing images of glioblastoma cell lines treated with selected cannabis extracts comprising various THC to CBD ratio. In this study, 10,000 U87MG glioblastoma cells were plated into a 96-well microplate and treated with vehicle (DMSO) or cannabis extracts. It is noted that the extracts were delectated to reach 5μM THC. Cells were imaged after 24 hours using the ImageXpress micro. It can be seen from the results that CBD plays a role in reducing U87MG glioblastoma cells viability.

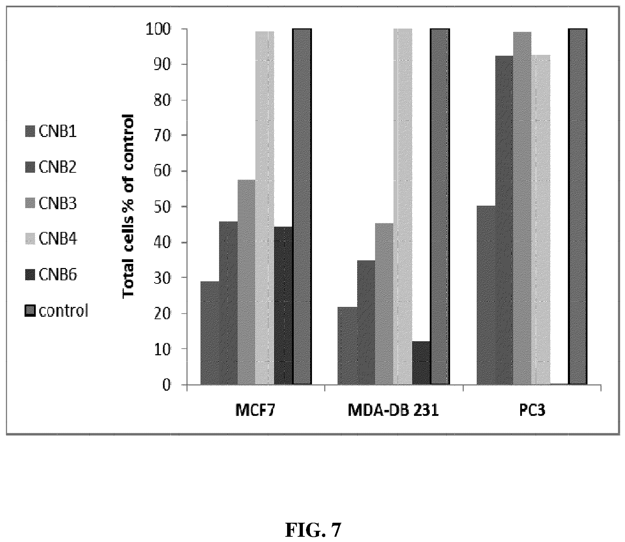

Reference is now made to **Fig. 7** graphically presenting the effect of five different cannabis extracts on three human cancer cell line types. More specifically, in this experiment, MCF-7, MDA-MB-231 (breast carcinoma) and PC3 (prostate carcinoma) cells were plated into a 24-well microplate and treated with vehicle (DMSO) as a control, or with cannabis extracts (10 μg/ml). 24 hours after treatment, cells were stained with Hoechst 33342, imaged using the ImageXpress micro and analyzed using MetaXpress. It is seen from the results that cannabis extracts have potent effect on cancer cells viability. The results further demonstrate the specificity of the different cannabis extracts tested against cell lines of various cancer types.

The results described above clearly demonstrate that the system and method of the current invention could be used to screen and select for cannabis extracts or fractions thereof with in vitro cytotoxicity towards cancer cells and minimal cytotoxicity towards normal cells. The cannabis extracts or fractions thereof that have been screened in this way can be further investigated for potential anti-tumor activity.

It is clearly shown that the HTS method and system of the present invention could differentiate cannabis plant or other plant extract's potential antitumor effects. It is shown that different strains cause varied apoptotic effects on different cancer cell lines with no effect on normal cells.

It is clear that the aforementioned potentially anti tumour extracts could be used as a basis for novel anti cancer compositions and therapies. Such compositions offer significant improvements to the currently available treatments against cancer in the following aspects:

- The present invention provides data on cannabis strains, cannabinoid ratios and /or plant genes that have potent effect on specific tumors.
- They could be administered to patients as part of a treatment regime, with or without chemotherapy at various stages of disease.
- Since cannabis extracts are purely natural they could be administered to patients with no regulatory procedures.
- Biopsies derived from patients could be scanned in the same procedure described *inter alia* and used as an important component in personalized medicine regimes.
- The provision of potent cannabinoid ratios could be used for the development of new botanical or synthetic drugs for treatment of specific types of cancer with or without combination of conventional antitumor drugs.
- Biological mechanisms could be revealed due to the analysis of active compounds and their binding receptors in the cells.

EXAMPLE 4

**Assessing antitumor activity of cannabis extracts on tumor xenografts**

In this example, the cancer cells lines which have shown *in-vitro* measurable effect on cells such as apoptosis and /or necrosis effects, as a result of treatment with specific compounds or analytes (e.g. cannabis extracts, see Example 3) are implanted in experimental animal, such as mice. The implanted mice are than treated with the selected analytes (such as extracts or compound or compounds combinations), with or without chemotherapy treatment as means for better evaluation of the effect of the selected analyte on cancer in humans.

Reference is now made to a xenograft tumor assay exemplified protocol **(https://www.mcdb.ucla.edu/Research/Arispe/Protocols/Xenograft Tumor Assay.pdf** is incorporated herein by its entirety). It is noted that modifications of this protocol are included within the scope of the present invention.

1) Determine the number of cells for injection (i.e. $5*10^6$) to determine the number of plates that will require trypsinizing (usually a 100% confluent plate of $100mm^2$ will yield at least 2 injections at $5*10^6$ cells/injection)

2) Trypsinize the number of plates to be counted all at once

3) Collect detached cells in 50 ml conical and spin for 4min at 800 rpm

4) Remove sup and resuspend in 25 ml of SFM for counting

5) Remove three 100 $\mu$l aliquots into 3 separate eppendorfs and dilute each 100 $\mu$l 1:5 by adding 400u. of SFM, mix well

6) Remove 50 $\mu$l of 1:5 dilutions for counting, count each of three dilutions and average the three numbers

7) Determine the conc. of cells in cells/ml by using the following formula:

$$\text{Average counts} *10,000 *\text{dilution factor (5)} = \#\text{cells/ml}$$

8) Determine the volume required to add to achieve final concentration of cells for injection per volume to be injected (i.e. 5*10+ cells/ 100 $\mu$l injection) by first determining the total number of cells in the 25 ml suspension by multiplying the conc. of cells in #cells/ml * 25 = total number of cells. Then use the following formula

$$\text{Total \# cells/x volume} = 5*10^6 / 100\mu l, \text{ solve for x = volume to resuspend pellet of cells to}$$

$$\text{achieve desired final concentration (i.e. } 5*10^6 \text{ cells/ } 100 \text{ } \mu l)$$

9) Spin down 50 ml conical for 4 min at 800 rpm

10) Discard sup and resuspend the pellet in the previously determined volume from step #8.

11) Draw up each injection/ mouse in 1 ml syringes in the tissue culture hood prior to going to the animal facility. Place the separate syringes each containing 100 $\mu$l on ice (this step minimized the possibility of the cells settling after being resuspended thus altering the concentration of cells.

12) Anesthetize each mouse with isoflorane inhalent just prior to injection. Be careful not to over anesthetize as the mice will succumb to respiratory depression. Just the right amount is when they just begin to stop moving, remove them from the source of anesthetic, let them breath pure air for a few seconds then place their noses just adjacent to the opening of the 50 ml conical duing the injection

13) Inject

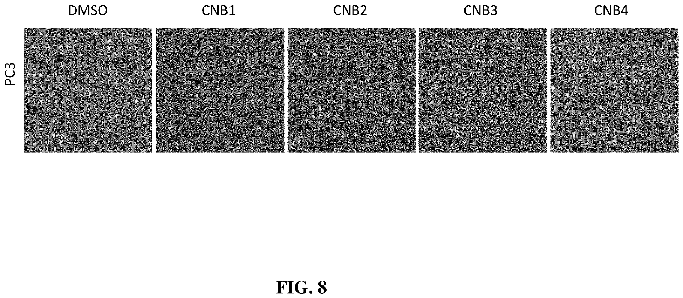

Reference is now made to **Fig. 8,** showing the effect of selected cannabis extracts on PC3 prostate carcinoma cells. In this experiment, at the first stage, PC3 prostate carcinoma cells were plated into a 24-well microplate and treated with vehicle (DMSO) or with selected cannabis extracts (10 $\mu$g/ml). Cells were imaged after 24 hours using the ImageXpress$^{micro}$. At the next stage, PC3 cells have been transplanted in nude mice for creating xenographs. The transplanted mice have been treated with selected cannabis extracts (i.e. CNB1, CNB2, CNB3 and CNB4). It can be seen from the images of **FIG. 8** that treating mice with CNB1 resulted in reduced tumor size, while treatment with CNB4 had no effect on the tumor.

EXAMPLE 5

**A scheme for identifying antitumor activity of selected cannabis extracts or fraction thereof**

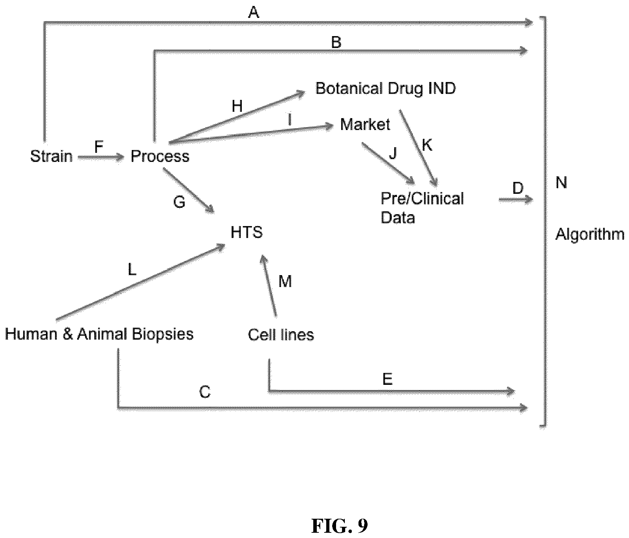

Reference is now made to **Fig. 9** presenting a scheme illustrating a protocol for identifying a botanical analyte with a measurable effect on cells correlated with a disease. In this figure, the biological activity of plant extracts (F) is screened *in vitro* (G) against cell lines (M) and/ or biopsies (L) for their biological activity. Data received from all process levels and parameters, including data concerning parameters of the analyte (A, B), parameters of the tested cells , high throughput screening (HTS) results data for identifying an analyte with a measurable effect on cells (C, E), clinical or preclinical data (D) and any combination thereof is being processed and organized by big data algorithms (N). Beneficial extracts are either being analyzed and active compound are listed as new IND (H) or directly given to patients as natural treatment (I). The data from case studies and clinical trials (J, K) creates a growing bank of extracts and botanical analytes that are targeted for specific ailments or cancers or mutations.

Reference is now made to Table 1 presenting some of the parameters used in the present invention for evaluation of antitumor activity of selected botanical extract or analyte.

**Table 1:** parameters for evaluation of antitumor activity of selected botanical extract or analyte

| Source | Process (compound or analyte) | Treatment | Cells (Source) | High Through output screening | Pre/Clinical Data |
|---|---|---|---|---|---|
| Strain: Genotype/ Phenotype | Curing - Time | Medium | Biopsies: Human Mammalian | Proliferation | Administration: Dosage /Sublingual Oral/ Intravenous/ Topical/ Subcutaneous |
| Light Time/ lumen | Extraction: Butane/$CO_2$- gradients/Ethan ol/Dry ice | Serum | Cell lines: Cancers/ Specific Mutations | Apoptosis | Cancer markers (See Example 6) |
| Time Harvest | Decarboxylatio n (Time/Temp) | Compound | | Migration | Tumor Size/Metastasis MRI/CT |
| Nutrition | | Dilution: DMSO/ Etha nol | | | Survival: Time |
| Plant Part: Root/ stem/flo wer/leaf | | Cell cycle Phase | | | Quality of Life: Pain, activity etc. |

EXAMPLE 6

Reference is now made to non limiting examples of cancer markers which may be used in the present invention for evaluation of antitumor activity of selected botanical extract or any other analyte.

ALK gene rearrangements

- Cancer types: Non-small cell lung cancer and anaplastic large cell lymphoma

- Tissue analyzed: Tumor

- How used: To help determine treatment and prognosis

Alpha-fetoprotein (AFP)

- Cancer types: Liver cancer and germ cell tumors

- Tissue analyzed: Blood

- How used: To help diagnose liver cancer and follow response to treatment; to assess stage, prognosis, and response to treatment of germ cell tumors

Beta-2-microglobulin (B2M)

- Cancer types: Multiple myeloma, chronic lymphocytic leukemia, and some lymphomas

- Tissue analyzed: Blood, urine, or cerebrospinal fluid

- How used: To determine prognosis and follow response to treatment

Beta-human chorionic gonadotropin (Beta-hCG)

- Cancer types: Choriocarcinoma and testicular cancer

- Tissue analyzed: Urine or blood

- How used: To assess stage, prognosis, and response to treatment

BCR-ABL fusion gene

- Cancer type: Chronic myeloid leukemia

- Tissue analyzed: Blood and/or bone marrow

- How used: To confirm diagnosis and monitor disease status

BRAF mutation V600E

- Cancer types: Cutaneous melanoma and colorectal cancer

- Tissue analyzed: Tumor

- How used: To predict response to targeted therapies

CA15-3/CA27.29

- Cancer type: Breast cancer

- Tissue analyzed: Blood

- How used: To assess whether treatment is working or disease has recurred

CA19-9

- Cancer types: Pancreatic cancer, gallbladder cancer, bile duct cancer, and gastric cancer

- Tissue analyzed: Blood

- How used: To assess whether treatment is working

CA-125

- Cancer type: Ovarian cancer

- Tissue analyzed: Blood

- How used: To help in diagnosis, assessment of response to treatment, and evaluation of recurrence

Calcitonin

- Cancer type: Medullary thyroid cancer

- Tissue analyzed: Blood

- How used: To aid in diagnosis, check whether treatment is working, and assess recurrence

Carcinoembryonic antigen (CEA)

- Cancer types: Colorectal cancer and breast cancer

- Tissue analyzed: Blood

- How used: To check whether colorectal cancer has spread; to look for breast cancer recurrence and assess response to treatment

CD20

- Cancer type: Non-Hodgkin lymphoma

- Tissue analyzed: Blood

- How used: To determine whether treatment with a targeted therapy is appropriate

Chromogranin A (CgA)

- Cancer type: Neuroendocrine tumors

- Tissue analyzed: Blood

- How used: To help in diagnosis, assessment of treatment response, and evaluation of recurrence

Chromosomes 3, 7, 17, and 9p21

- Cancer type: Bladder cancer

- Tissue analyzed: Urine

- How used: To help in monitoring for tumor recurrence

Cytokeratin fragments 21-1

- Cancer type: Lung cancer

- Tissue analyzed: Blood

- How used: To help in monitoring for recurrence

EGFR mutation analysis

- Cancer type: Non-small cell lung cancer

- Tissue analyzed: Tumor

- How used: To help determine treatment and prognosis

Estrogen receptor (ER)/progesterone receptor (PR)

- Cancer type: Breast cancer

- Tissue analyzed: Tumor

- How used: To determine whether treatment with hormonal therapy (such as tamoxifen) is appropriate

Fibrin/fibrinogen

- Cancer type: Bladder cancer

- Tissue analyzed: Urine

- How used: To monitor progression and response to treatment

HE4

- Cancer type: Ovarian cancer

- Tissue analyzed: Blood

- How used: To assess disease progression and monitor for recurrence

HER2/neu

- Cancer types: Breast cancer, gastric cancer, and esophageal cancer

- Tissue analyzed: Tumor

- How used: To determine whether treatment with trastuzumab is appropriate

Immunoglobulins

- Cancer types: Multiple myeloma and Waldenstrom macroglobulinemia

- Tissue analyzed: Blood and urine

- How used: To help diagnose disease, assess response to treatment, and look for recurrence

KIT

- Cancer types: Gastrointestinal stromal tumor and mucosal melanoma

- Tissue analyzed: Tumor

- How used: To help in diagnosing and determining treatment

KRAS mutation analysis

- Cancer types: Colorectal cancer and non-small cell lung cancer

- Tissue analyzed: Tumor

- How used: To determine whether treatment with a particular type of targeted therapy is appropriate

Lactate dehydrogenase

- Cancer type: Germ cell tumors

- Tissue analyzed: Blood

- How used: To assess stage, prognosis, and response to treatment

Nuclear matrix protein 22

- Cancer type: Bladder cancer
- Tissue analyzed: Urine
- How used: To monitor response to treatment

Prostate-specific antigen (PSA)

- Cancer type: Prostate cancer
- Tissue analyzed: Blood
- How used: To help in diagnosis, assess response to treatment, and look for recurrence

Thyroglobulin

- Cancer type: Thyroid cancer
- Tissue analyzed: Tumor
- How used: To evaluate response to treatment and look for recurrence

Urokinase plasminogen activator (uPA) and plasminogen activator inhibitor (PAI-1)

- Cancer type: Breast cancer
- Tissue analyzed: Tumor
- How used: To determine aggressiveness of cancer and guide treatment

5-Protein signature (Oval)

- Cancer type: Ovarian cancer
- Tissue analyzed: Blood
- How used: To pre-operatively assess pelvic mass for suspected ovarian cancer

21-Gene signature (Oncotype DX)

- Cancer type: Breast cancer
- Tissue analyzed: Tumor
- How used: To evaluate risk of recurrence

70-Gene signature (Mammaprint)

- Cancer type: Breast cancer
- Tissue analyzed: Tumor
- How used: To evaluate risk of recurrence

**Claims**

1. A database of analytes, wherein said database comprises data concerning said analyte, correlated with a measurable effect on cells, defined by implementing personalized medicine PM based method comprising high throughput screening HTS for identifying antitumor effect of cannabis strains comprising the steps of:

   a. providing an array comprising a plurality of tumor cell samples of a patient's tumor biopsy;
   b. providing at least one cannabis extract of at least one strain to be tested;
   c. contacting said patient's tumor biopsy tumor cell samples with said at least one cannabis extract; and
   d. detecting a signal indicative of said antitumor effect relative to a control sample.

**2.** The database according to claim 1, additionally comprises at least one step of:

  a. correlating biological data of said patient from which said biopsy is derived with the antitumor effect data from said HTS method;
  b. correlating the clinical data of the patient from which said biopsy is derived with the antitumor effect data from said HTS method.

**3.** The database according to claim 2, wherein said biological data is selected from the group consisting of: genetic, blood, neurology, behavior, nutrition and combinations thereof.

**4.** The database according to claim 1, wherein at least one of the following holds true:

  a. said step of contacting comprises contacting said patient's biopsy derived cells with said at least one cannabis extract and with at least one conventional chemotherapy drug;
  b. said antitumor effect is selected from the group consisting of: anti proliferative, regenerative, anti inflammatory, anti mitotic, differentiative, anti metastatic, anti angiogenic, apoptotic, cytotoxic, cytopathic and any combination thereof.
  c. said antitumor effect is selected from the group consisting of physiological, genetic, biochemical, structural and any combination thereof;
  d. said biopsy derived cells are selected from the group consisting of human cells, animal cells and xenografts;
  e. said biopsy derived cells are selected from the group consisting of: cancer cells, stem cells, neuronal cells, cardiomyocyte cells, somatic cells, germ cells, normal cells, and any combination thereof;
  f. said strain is subjected to defined source growth conditions, source harvest conditions and extraction process;
  g. said tumor biopsy derived cells are selected from the group consisting of : breast, ovarian, colon/ rectum, prostate, melanoma, head and neck, pancreatic, osteosarcoma, gastric, glioma, autonomic ganglia, glioblastoma, neuroblastoma, leukemia, adenocarcinoma, adrenal, anal, bile duct, bladder, bone, brain/CNS, cervical, endometrial, esophagus, eye, gastrointestinal, kidney, leukemia, liver, lung , lymphoma, multiple myeloma, nasal cavity and paranasal sinus, nasopharyngeal, non-hodgkin lymphoma, oral cavity, oropharyngeal, osteosarcoma, ovarian, pancreatic, penile, pituitary, retinoblastoma, rhabdomyosarcoma, salivary gland, sarcoma, skin, small intestine, large intestine, stomach, testicular, thymus, thyroid, uterine sarcoma, urinary tract vaginal, ovary, haematopoietic and lymphoid tissue, soft tissue, pleura, endometrium, pancreas, upper aerodigestive tract, oesophagus, biliary tract, vulvar and any combination thereof;
  h. said signal is selected from the group consisting of: optic, luminescent, fluorescent, immunological, cell count, radioactive, non radioactive isotopic, electrical and any combination thereof;
  i. said extract is derived from a cannabis species selected from a group consisting of: Cannabis sativa, Cannabis indica, Cannabis ruderalis, and any combination thereof.
  j. said HTS is selected from the group consisting of: microtiter plate, automatic colony pickers, uHTS or ultra-high-throughput screening, 3D tumor spheroid analysis method for HTS drug discovery, Celigo Imaging Cytometer, automation systems, a carousel system to store assay plates for high storage capacity and high speed access, integrated robot system, readout or detection, data-collection process and any combination thereof.

**5.** The database of claim 4, wherein said extract provides a synergistic effect with respect to said antitumor effect as compared to the effect provided by conventional antitumor or anti- inflammatory therapies administered separately.

**6.** The database of claim 5, wherein said extract provides a contra indicatory effect with respect to antitumor or anti-inflammatory activity as compared to the effect provided by conventional antitumor or anti-inflammatory therapies administered separately.

**7.** A database comprising data collected by a high throughput screening HTS method for identifying antitumor effect of cannabis strains; said method comprising steps of:

  a. providing an array comprising a plurality of tumor cell samples of a patient's tumor biopsy;
  b. providing at least one cannabis extract of at least one strain to be tested;
  c. contacting said patient's tumor biopsy tumor cell samples with said at least one cannabis extract; and
  d. detecting a signal indicative of said antitumor effect relative to a control sample.

**8.** The database according to claim 7 wherein the method additionally comprises at least one step of:

a. correlating biological data of said at least one patient from which said biopsy is derived with the antitumor effect data from said HTS method;

b. correlating the clinical data of said at least one patient from which said biopsy is derived with the antitumor effect data from said HTS method.

| | Form No F86 |
|---|---|
| Test Report for Cannabinoid Analysis – ISO 17025 | |
| Page 1 of 1 | Version 1 – 01Jan2014 |

**izun** Pharma Ltd

ilac-MRA

ISRAC
הרשות הלאומית להסמכת מעבדות
ISO/IEC 17025
No. **308** סמ

## Test Report

**Report No.:** RPB 09-15                    **Date:** 010May2015

**Test Method:** *Preparation of Cannabis Oil and Analysis by HPLC* Form No. F90

**Customer name:** Cannabics Pharmaceuticals          **Address:**

**Contact person:** Dr. Eyal Ballan               **Telephone number:** 050-8619215

**E-mail:** eyal@cannabics.com                **Quotation No.:** 012-15

**Receipt of sample:** 06May2015              **Record No.:** F90-07-15

**Sampling and transportation is the responsibility of the customer.**

### Results (based on dry weight of total sample received)

| Sample ID | | Cannabinoid Constituent (% w/w) | | | | | | | | | | | |
|---|---|---|---|---|---|---|---|---|---|---|---|---|---|
| Customer | Izun | CBDA | CBGA* | CBG | CBD | THCV* | CBN | THCA | Δ9THC | Δ8THC | CBC | Total THC | Total CBD |
| CNB1 | cc-018-15 | 0.3 | 1.8 | 0.6 | ND | ND | 0.1 | 82.2 | 1.2 | ND | <0.1 | 73.4 | 0.2 |
| CNB2 | cc-019-15 | 0.7 | 3.3 | ND | <0.1 | ND | 0.3 | 71.3 | 5.8 | ND | 0.2 | 68.4 | 0.7 |
| CNB3 | cc-020-15 | 70.2 | 0.7 | ND | 10.7 | <0.1 | 0.1 | 2.1 | 2.1 | ND | 0.9 | 4.0 | 72.4 |
| CNB4 | cc-021-15 | 0.4 | 1.0 | 0.8 | ND | <0.1 | 1.7 | 36.1 | 41.9 | ND | 0.5 | 73.5 | 0.3 |
| CNB6 | cc-022-15 | 0.3 | 0.7 | 0.9 | ND | <0.1 | 2.6 | 55.1 | 16.0 | ND | 0.4 | 64.4 | 0.3 |
| CNB8 | cc-023-15 | <0.1 | 0.1 | 4.6 | ND | 0.4 | 1.0 | 0.1 | 78.2 | 1.0 | 0.7 | 78.3 | <0.1 |

Comments: *Analysis of THCV and CBGA are not under ISO/IEC 17025 certification.

Analyzed by: Jenna Mintz, Senior Chemist          Signature _IZUN PHARMA LTD_

Approved by: Dr. Shmuel Cohen               Signature _(for S Cohen)_

### The laboratory is accredited under ISO/IEC 17025

This certificate is valid only when presented in its complete format, and it is not permitted to extract part for inclusion in any other document without written consent of Izun Pharma Ltd.

The laboratory is authorized by the Laboratory Accreditation Authority. The Authority is not responsible for the above results and the authorization does not represent authorization of the parameters reported.

\*\*\*End of report\*\*\*

P.O.Box 45088 Jerusalem, 91450 ♦ Tel: 972-2-586-0780 ♦ Fax: 972-2-586-1814
info@izunpharma.com

## FIG. 1

**FIG. 2**

**FIG. 3A**

**FIG. 3B**

**FIG. 3C**

**FIG. 4**

DMSO CNB1 CNB2 CNB3 CNB4

PC3

**FIG. 5**

DMSO          20%THC 7%CBD          20%THC 0.12%CBD

U87MG

**FIG. 6**

**FIG. 7**

**FIG. 8**

FIG. 9

Europäisches
Patentamt

European
Patent Office

Office européen
des brevets

## EUROPEAN SEARCH REPORT

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 2 044 935 A1 (VIVACELL BIOTECHNOLOGY ESPANA [ES]) 8 April 2009 (2009-04-08) * the whole document * * in particular: * * examples 3-8 * ----- | 1-8 | INV. G01N33/50 G01N33/574 A61P29/00 A61P35/00 A61P43/00 G01N33/94 |
| A | LUCIANO DE PETROCELLIS ET AL: "Non-THC cannabinoids inhibit prostate carcinoma growth in vitro and in vivo : pro-apoptotic effects and underlying mechanisms", BRITISH JOURNAL OF PHARMACOLOGY, vol. 168, no. 1, 18 December 2012 (2012-12-18), pages 79-102, XP055335428, UK ISSN: 0007-1188, DOI: 10.1111/j.1476-5381.2012.02027.x * the whole document * * in particular: * * par. EXPERIMENTAL APPROACH; page 79 * * Methods; page 81 – page 84 * * figures 1,3,4 * ----- | 1-8 | |
| A | WO 2012/050981 A1 (MASSACHUSETTS INST TECHNOLOGY [US]; KAMM ROGER DALE [US]; ASADA HARUHI) 19 April 2012 (2012-04-19) * the whole document * * in particular: * * page 16, line 7 – line 10 * * page 45, line 15 – line 21 * ----- | 1-8 | TECHNICAL FIELDS SEARCHED (IPC) G01N A61P |
| A | US 2013/316392 A1 (ANANT SHRIKANT [US] ET AL) 28 November 2013 (2013-11-28) * paragraph [0031] * ----- | 1-8 | |

-/--

The present search report has been drawn up for all claims

| Place of search | Date of completion of the search | Examiner |
|---|---|---|
| The Hague | 5 January 2022 | Tuynman, Antonin |

EPO FORM 1503 03.82 (P04C01)

**page 1 of 2**

Europäisches
Patentamt

European
Patent Office

Office européen
des brevets

# EUROPEAN SEARCH REPORT

Application Number

EP 21 17 2650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2010/209959 A1 (MIRSHAHI MASSOUD [FR] ET AL) 19 August 2010 (2010-08-19) * the whole document * ----- | 1-8 | |
| A | US 2008/262099 A1 (WHITTLE BRIAN [GB] ET AL) 23 October 2008 (2008-10-23) * the whole document * ----- | 1-8 | |

TECHNICAL FIELDS
SEARCHED (IPC)

The present search report has been drawn up for all claims

| Place of search | Date of completion of the search | Examiner |
|---|---|---|
| The Hague | 5 January 2022 | Tuynman, Antonin |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**EP 3 954 992 A1**

## ANNEX TO THE EUROPEAN SEARCH REPORT
## ON EUROPEAN PATENT APPLICATION NO.

EP 21 17 2650

This annex lists the patent family members relating to the patent documents cited in the above-mentioned European search report.
The members are as contained in the European Patent Office EDP file on
The European Patent Office is in no way liable for these particulars which are merely given for the purpose of information.

05-01-2022

| Patent document cited in search report | | Publication date | Patent family member(s) | | Publication date |
|---|---|---|---|---|---|
| EP 2044935 | A1 | 08-04-2009 | AT | 493977 T | 15-01-2011 |
| | | | CA | 2701368 A1 | 09-04-2009 |
| | | | EP | 2044935 A1 | 08-04-2009 |
| | | | ES | 2359168 T3 | 19-05-2011 |
| | | | JP | 2010540587 A | 24-12-2010 |
| | | | US | 2010222437 A1 | 02-09-2010 |
| | | | WO | 2009043836 A1 | 09-04-2009 |
| WO 2012050981 | A1 | 19-04-2012 | CA | 2813211 A1 | 19-04-2012 |
| | | | CN | 103477222 A | 25-12-2013 |
| | | | EP | 2622342 A1 | 07-08-2013 |
| | | | JP | 6170431 B2 | 26-07-2017 |
| | | | JP | 2013538582 A | 17-10-2013 |
| | | | KR | 20130131326 A | 03-12-2013 |
| | | | SG | 189160 A1 | 31-05-2013 |
| | | | SG | 10201508047S A | 29-10-2015 |
| | | | US | 2014057311 A1 | 27-02-2014 |
| | | | WO | 2012050981 A1 | 19-04-2012 |
| US 2013316392 | A1 | 28-11-2013 | US | 2013316392 A1 | 28-11-2013 |
| | | | WO | 2013177011 A2 | 28-11-2013 |
| US 2010209959 | A1 | 19-08-2010 | CA | 2691985 A1 | 15-01-2009 |
| | | | EP | 2162532 A2 | 17-03-2010 |
| | | | FR | 2918073 A1 | 02-01-2009 |
| | | | JP | 2010531150 A | 24-09-2010 |
| | | | US | 2010209959 A1 | 19-08-2010 |
| | | | WO | 2009007618 A2 | 15-01-2009 |
| US 2008262099 | A1 | 23-10-2008 | AT | 408412 T | 15-10-2008 |
| | | | CA | 2582289 A1 | 13-04-2006 |
| | | | DK | 1802274 T3 | 02-02-2009 |
| | | | EP | 1802274 A1 | 04-07-2007 |
| | | | ES | 2313414 T3 | 01-03-2009 |
| | | | GB | 2418612 A | 05-04-2006 |
| | | | JP | 6280489 B2 | 14-02-2018 |
| | | | JP | 2008514687 A | 08-05-2008 |
| | | | JP | 2012131803 A | 12-07-2012 |
| | | | JP | 2014094960 A | 22-05-2014 |
| | | | JP | 2015038137 A | 26-02-2015 |
| | | | JP | 2017031190 A | 09-02-2017 |
| | | | US | 2008262099 A1 | 23-10-2008 |
| | | | US | 2012225136 A1 | 06-09-2012 |
| | | | US | 2019255011 A1 | 22-08-2019 |
| | | | WO | 2006037981 A1 | 13-04-2006 |

EPO FORM P0459

For more details about this annex : see Official Journal of the European Patent Office, No. 12/82

40